# EUROPEAN PATENT SPECIFICATION

(11) **EP 0 487 357 B1**
(45) Date of publication and mention of the grant of the patent: **02.07.1997**
(21) Application number: 91310796.7
(22) Date of filing: 22.11.1991
(51) Int. Cl.: C07D 261/08, C07D 261/18, C07D 261/10, A01N 43/80

(54) **4 Benzoyl isoxazole derivatives**
4-Benzoylisoxazolderivate
Dérivés de 4-benzoyl isoxazoles

(30) Priority: 22.11.1990 GB 9025469
(43) Date of publication of application: 27.05.1992
(73) Proprietor: RHONE-POULENC AGRICULTURE LTD., Ongar, Essex CM5 0HW (GB)
(72) Inventor: Cain, Paul Alfred, Ongar Essex CM5 OHW (GB); Cramp, Susan Mary, Ongar Essex CM5 OHW (GB); Roberts, David Alan, Ongar Essex CM5 OHW (GB)
(74) Representative: Bentham, Stephen

(56) References cited:
- EP-A- 0 283 261
- EP-A- 0 418 175
- GB-A- 2 201 672
- Zhurnal Obs. Khim. (1960) 30(11), pp 3675-3682
- J. Het. Chem. (1975) 12, pp 49-57
- J. med. Chem. (1984) 27(10), pp 1245-1253
- Zhurnal Obs. Khim. (1964) 34(7), pp 2207-2209

## Description

This invention relates to 4-benzoyl isoxazole derivatives, processes for their preparation, compositions containing them and their use as herbicides.

GB-A-2201672 discloses inter alia substituted isoxazoles bearing a phenoxyphenyl substituent in the 3-position, which are said to possess herbicidal activity. EP-A-0283261 discloses certain herbicidal substituted cyclic diones, and in particular 4-substituted isoxazoles, wherein the 4-position bears a cyclohexadione-carbonyl moiety. EP-A-0418175, which was filed before the present invention but published after the priority date of the present invention, discloses herbicidally active 3-unsubstituted-4-benzoyl isoxazoles. Koehetkov et al, Zhurnal, Obs. Khim., Volume 30(11), pp3675-3682 (1960); Kalish et al, J. Het. Chem., Vol.12, pp49-57 (1975); Ashton et al, J. Med. Chem., Vol. 27, pp 1245-1253 (1984); and Sokolov et al, Zhurnal, Obs. Khim., Vol. 34(7), pp2207-2209 (1964) each describe certain 3,5-dimethyl-4-benzoyl isoxazole compounds; none of these references disclose any biological activity in these compounds.

The present invention provides the 4-benzoyl isoxazoles of the general formula I shown hereinafter in the present specification, wherein:
R represents :-
   a straight- or branched-chain alkyl, alkenyl or alkynyl group containing up to 6 carbon atoms which is optionally substituted by one or more halogen atoms; or
   a cycloalkyl group containing from 3 to 6 carbon atoms optionally substituted by one or more substituents selected from groups R⁵, one or more halogen atoms and a group -CO₂R³; or
   a group selected from -CO₂R³, -COR⁵, cyano, nitro, -CONR³¹R⁴,
   or a halogen atom;
R¹ represents :-
   a hydrogen atom, or
   a straight- or branched-chain alkyl, alkenyl or alkynyl group containing up to 6 carbon atoms which is optionally substituted by one or more halogen atoms; or
   a cycloalkyl group containing from 3 to 6 carbon atoms optionally substituted by one or more substituents selected from groups R⁵ and one or more halogen atoms;
R² represents :-
   a halogen atom, or
   a group selected from R⁵, -SR⁵, -SOR⁵, -SO₂R⁵, -SO₂NR³¹R⁴, -CO₂R³, -COR⁵, -CONR³¹R⁴, -CSNR³¹R⁴, -OR⁵, a nitro group, a cyano group, a group -O(CH₂)_{q}-OR⁵, or a straight- or branched- chain alkyl group containing up to 6 carbons and which is substituted by OR⁵;
R³, R³¹ and R⁴, which may be the same or different, each represents:-
   a hydrogen atom, or
   a straight- or branched-chain alkyl group containing up to 6 carbon atoms which is optionally substituted by one or more halogen atoms;
R⁵ represents :-
   a straight- or branched-chain alkyl group containing up to 6 carbon atoms which is optionally substituted by one or more halogen atoms;
n represents an integer from 1 to 5;
q represents an integer from 1 to 3;
provided that R and R¹ do not simultaneously represent a methyl group;
and agriculturally acceptable salts thereof, which possess valuable herbicidal properties.

When n represents an integer from 2 to 5, the substituents R² may be the same or different.

Furthermore, in certain cases the substituents R, R¹, R², R³, R³¹, R⁴ and R⁵ contribute to optical isomerism and/or stereoisomerism. All such forms are embraced by the present invention.

By the term "agriculturally acceptable salts" is meant salts, the cations of which are known and accepted in the art for the formation of salts for agricultural or horticultural use. Preferably the salts are water-soluble.

Suitable salts formed by compounds of formula I which are acidic, i.e. compounds containing one or more carboxy groups, with bases include alkali metal (e.g sodium and potassium) salts, alkaline earth metal (e.g. calcium and magnesium) salts, ammonium and amine (e.g diethanolamine, triethanolamine, octylamine, dioctylmethylamine and morpholine) salts.

It is to be understood that where reference is made in the present specification to the compounds of general formula I, such reference is intended to include salts where the context so permits.

Herbicidal compositions comprising a compound of general formula I wherein R and R¹ simultaneously represent a methyl group and the use of such compounds for the control of the growth of weeds constitute part of the invention as hereafter defined.

Particularly important classes of compounds of formula I because of their herbicidal properties include those which exhibit one or more of the following features : -
(a) R represents :-
   a straight- or branched- chain alkyl group containing up to 6 carbon atoms which is optionally substituted by one or more halogen atoms, for example a methyl, ethyl, isopropyl or trifluoromethyl group; or
   a cycloalkyl group containing from 3 to 6 carbon atoms, for example a cyclopropyl group, or
   a group selected from -COR⁵, -CO₂R³, or a halogen atom, preferably a bromine atom ;
(b) R¹ represents :-
   a straight- or branched- chain alkyl group containing up to 6 carbon atoms, for example a methyl group or isopropyl group, or
   a cycloalkyl group of 3 or 4 carbon atoms which is optionally substituted by one or more R⁵ groups, for example cyclopropyl or 1-methylcyclopropyl ;
(c) R² represents :-
   a halogen atom, particularly chlorine, fluorine or bromine, or
   an alkyl group containing up to 6 carbons which is substituted by - OR⁵;
   or a group selected from R⁵, nitro, -CO₂R³, -SOR⁵, -SR⁵, -SO₂R⁵ and -OR⁵;
(d) at least one group R² occupies an ortho position adjacent to the carbonyl group linking the phenyl and isoxazolyl rings;
(e) R³ represents :-
   a straight or branched-chain alkyl group containing up to 6 carbon atoms, for example a methyl or ethyl group;
(f) R⁴ represents a hydrogen atom ;
(g) R⁵ represents a straight- or branched-chain alkyl group containing up to 6 carbon atoms which is optionally substituted by one or more halogen atoms, for example a methyl or trifluoromethyl group ;
   the other symbols being as bereinbefore defined, and agriculturally acceptable salts thereof.

A further preferred class of compounds of formula I are those which include one or more of the following features:
R represents a group -CO₂R³ or -COR⁵, most preferably -CO₂R³;
R¹ represents a straight- or branched- chain alkyl group containing up to 6 carbon atoms, e.g. methyl, ethyl, isopropyl, or a cycloalkyl group containing 3 or 4 carbon atoms optionally substituted by one or more groups R⁵, e.g. cyclopropyl or 1-methylcyclopropyl;
R² represents a halogen atom, preferably chlorine or bromine; or
   an alkyl group containing up to 6 carbon atoms which is substituted by -OR⁵; or
   a group R⁵, - NO₂, -CO₂R³, -SR⁵, SOR⁵, - SO₂R⁵, -OR⁵, or
   a group -O(CH₂)_{q}·OR⁵;
R³ represents a straight- or branched- chain alkyl group containing up to 6 carbon atoms;
R³¹ represents hydrogen; R⁴ represents hydrogen;
R⁵ represents a straight- or branched- chain alkyl group containing up to 3 carbon atoms optionally substituted by one or more halogen atoms;
   one of the groups R² is in the ortho position of the phenyl ring;
   and n represents 2 or 3.

Compounds of formula I which are of particular interest include the following :-
A) 3-Methyl-4-(2-nitro-4-trifluoromethylbenzoyl)-isoxazole;
B) 3-(1-Methylethyl-4-(2-nitro-4-trifluoromethylbenzoyl)-isoxazole;
C) Ethyl 4-(2-nitro-4-trifluoromethylbenzoyl)-isoxazole-3-carboxylate;
D) 3-Bromo-4-(2-nitro-4-trifluoromethylbenzoyl)-isoxazole;
E) 4-(2-Nitro-4-trifluoromethylbenzoyl)-3-trifluoromethylisoxazole;
F) 5-Cyclopropyl-3-methyl-4-(2-nitro-4-trifluoromethylbenzoyl)-isoxazole;
G) Ethyl 5-methyl-4-(2-nitro-4-trifluoromethylbenzoyl)-isoxazole-3-carboxylate;
H) 5-Methyl-4-(2-nitro-4-trifluoromethylbenzoyl)-isoxazole-3-carboxamide;
I) Ethyl 5-cyclopropyl-4-(2-nitro-4-trifluoromethylbenzoyl)-isoxazole-3-carboxylate;
J) 3-Cyano-5-methyl-4-(2-nitro-4-trifluoromethylbenzoyl)-isoxazole;
K) Ethyl 4-(2-chloro-4-methylsulphonylbenzoyl)-5-cyclopropylisoxazole-3-carboxylate;
L) Ethyl 5-cyclopropyl-4-(4-methylsulphonyl-2-trifluoromethylbenzoyl) isoxazole-3-carboxylate;
M) Methyl 4-(2-chloro-4-methylsulphonylbenzoyl)-5-cyclopropylisoxazole-3-carboxylate.
N) 4-(2-chloro-4-methylsulpbonylbenzoyl)-5-cyclopropyl-isoxazole-3-carboxamide;
O) Ethyl 5-cyclopropyl-4-(2-methylsulphonylbenzoyl)-isoxazole-3-carboxylate;
P) Methyl 5-cyclopropyl-4-(4-methylsulphonyl-2-trifluoromethylbenzoyl)-isoxazole-3-carboxylate;
Q) Ethyl-5-cyclopropyl-4-(2-nitro-4-methylsulphonylbenzoyl)-isoxazole-3-carboxylate;
R) Ethyl 5-cyclopropyl-4-(2,3-dichloro-4-methylsulphonylbenzoyl)-isoxazole-3-carboxylate;
S) Ethyl 4-(2-chloro-3-methoxy-4-methylsulphonylbenzoyl)-5-cyclopropylisoxazole-3-carboxylate;
T) 4-(2-chloro-4-methylsulphonylbenzoyl)-3-cyano-5-cyclopropylisoxazole;
U) Ethyl 4-(4-chloro-2-methylsulphonylbenzoyl)-5-cyclopropylisoxazole-3-carboxylate;
V) Ethyl 4-(4-chloro-2-nitrobenzoyl)-5-cyclopropylisoxazole-3-carboxylate;
W) Ethyl 4-(4-chloro-2-trifluoromethylbenzoyl)-5-cyclopropylisoxazole-3-carboxylate;
X) Ethyl 5-cyclopropyl-4-[2-methylsulphenyl-4-trifluoromethylbenzoyl]-isoxazole-3-carboxylate;
Y) Ethyl 5-cyclopropyl-4-[4-methylsulphenyl-2-trifluoromethylbenzoyl]-isoxazole-3-carboxylate;
Z) Ethyl 5-cyclopropyl-4-(2-methylsulphinyl-4-trifluoromethylbenzoyl)-isoxazole-3-carboxylate;
AA) Ethyl 5-cyclopropyl-4-(4-methylsulphinyl-2-trifluoromethylbenzoyl)-isoxazole-3-carboxylate;
BB) Ethyl 5-cyclopropyl-4-(2-methylsulphonyl-4-trifluoromethylbenzoyl)-isoxazole-3-carboxylate;
CC) Ethyl 5-cyclopropyl-4-(2-fluoro-4-methylsulphonylbenzoyl)-isoxazole-3 -carboxylate;
DD) Ethyl 4-(2-chloro-4-ethylsulphonylbenzoyl)-5-cyclopropylisoxazole-3-carboxylate;
EE) Ethyl 4-(4-bromo-2-methylsulphonylbenzoyl)-5-cyclopropylisoxazole-3-carboxylate;
FF) Ethyl 4-(2-bromo-4-methylsulphonylbenzoyl)-5-cyclopropylisoxazole-3-carboxylate;
GG) Ethyl 4-[2-chloro-4-methylsulphenyl-benzoyl]-5-cyclopropylisoxazole-3-carboxylate;
HH) Ethyl 4-(2-chloro-4-methylsulphinylbenzoyl)-5-cyclopropylisoxazole-3-carboxylate;
II) 3-Acetyl-4-(2-chloro-4-methylsulphonylbenzoyl)-5-cyclopropyl isoxazole;
JJ) Isopropyl 4-(2-chloro-4-methylsulphonylbenzoyl)-5-cyclopropylisoxazole-3-carboxylate;
KK) Methyl 4-(4-chloro-2-methylsulphonylbenzoyl)-5-cyclopropylisoxazole-3-carboxylate;
LL) Methyl 5-cyclopropyl-4-(2-methylsulphonyl-4-trifluoromethylbenzoyl)-isoxazole-3-carboxylate;
MM) Methyl 4-[4-chloro-2-methylsulphenyl-benzoyl]-5-cyclopropylisoxazole-3-carboxylate;
NN) Methyl 4-(4-chloro-2-methylsulphinylbenzoyl)-5-cyclopropylisoxazole-3-carboxylate.

The letters A to NN are assigned to the compounds for identification and reference hereinafter.

Compounds of general formula I can be prepared by the application or adaptation of known methods (i.e. methods heretofore used or described in the literature), for example methods as hereinafter described.

According to a feature of the present invention, compounds of general formula I wherein R, R¹, R² and n are as hereinbefore defined, may be prepared by the reaction of compounds of the general formula II shown hereinafter, wherein R and R¹ are as hereinbefore defined and X¹ represents a halogen (preferably a chlorine) atom, with compounds of the general formula III shown hereinafter, wherein R² and n are as hereinbefore defined, in the presence of a Lewis acid catalyst, for example aluminium chloride. The reaction is generally performed in an inert solvent at a temperature from 0°C to 100°C.

According to a further feature of the present invention compounds of general formula I wherein R, R¹, R² and n are as hereinbefore defined may be prepared by the reaction of compounds of the general formula IV shown hereinafter, wherein R¹, R² and n are as hereinbefore defined and X² represents a group of the formula -N(R⁷)₂ or -SR⁷, wherein R⁷ represents a straight- or branched- chain alkyl group of up to 4 carbon atoms, with compounds of the general formula V:-

RC(X¹) = N-OH V

wherein R and X¹ are as hereinbefore defined, in the presence of an organic base, for example triethylamine or pyridine, in an inert solvent, for example toluene. The reaction may also be carried out in the presence of a catalyst such as a molecular sieve or fluoride ion such as potassium fluoride in an inert solvent such as dichloromethane.

According to a further feature of the present invention compounds of general formula I wherein R, R¹, R² and n are as hereinbefore defined may be prepared by the reaction of compounds of the general formula VI show hereinafter, wherein R¹, R² and n are as hereinbefore defined, with compounds of formula V wherein R and X¹ are as hereinbefore defined, in the presence of an organic base or a catalyst in an inert solvent, such as dichloromethane. The reaction may be carried out in the presence of a catalyst, for example, a molecular sieve or fluoride ion such as potassium fluoride.

According to a further feature of the present invention compounds of general formula I wherein R, R¹, R², and n are as hereinbefore defined, may be prepared by the oxidation of compounds of general formula VII shown hereinafter, wherein R,R¹,R², and n are as hereinbefore defined, to convert the hydroxy group to a ketone group, for example by means of a mixture prepared from chromium trioxide, aqueous sulphuric acid and acetone.

According to a further feature of the present invention compounds of general formula I wherein R, R¹, R² and n are as hereinbefore defined may be prepared by metallation of compounds of general formula VIII shown hereinafter, wherein R and R¹ are as hereinbefore defined and X³ represents bromine or iodine, with for example n-butyllithium in an inert solvent such as diethyl ether or tetrahydrofuran at a temperature from -78°C to 0°C, followed by treatment with the benzoyl chloride of general formula IX shown hereinafter in which R² and n are as hereinbefore defined.

According to a further feature of the present invention compounds of general formula I wherein R, R¹, R² and n are as hereinbefore defined may be prepared by the reaction of a salt of compounds of general formula X shown hereinafter wherein R¹, R² and n are as hereinbefore defined, with a compound of general formula V. Generally the reaction is performed in an inert apolar solvent e.g. toluene from 0°C to 80°C. Preferred salts include sodium or magnesium salts.

Intermediates used in the preparation of compounds of general formula I may be prepared by the application or adaptation of known methods, for example methods described below.

Intermediates of general formula IV may be prepared by the reaction of an acetophenone of general formula XI shown hereinafter, wherein R² and n are as hereinbefore defined, with an amide acetal of general formula XII:

R¹-C(OR⁷)₂-X³ XII

wherein R¹ and R⁷ are as as hereinbefore defined and X³ represents a group of the formula -N(R⁷)₂, wherein R⁷ is as hereinbefore defined, optionally in the presence of an inert solvent such as toluene, at a temperature from room temperature to the reflux temperature of the mixture.

Alternatively, intermediates of general formula IV may be prepared by the reaction of an enamine of general formula XIII: 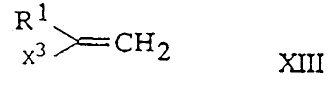 wherein R¹ and X³ are as hereinbefore defined, with a benzoyl chloride of general formula IX in the presence of an organic base such as triethylamine in an inert solvent such as toluene or dichloromethane at a temperature from -20°C to room temperature.

Intermediates of general formula VI may be prepared by metallation of the appropriate acetylene of general formula XIV:

R¹C≡CH XIV

wherein R¹ is as hereinbefore defined using for example n-butyllithium in an inert solvent such as ether or tetrahydrofuran at a temperature from -78° to 0°C, followed by reaction of the metal salt thus obtained with a benzoyl chloride of general formula IX.

Intermediates of general formula VII may be prepared by the metallation of compounds of general formula VIII with for example n-butyllithium in an inert solvent such as ether or tetrahydrofuran at a temperature from -78° to 0°C followed by treatment with the benzaldehyde of general formula XV shown hereinafter wherein R² and n are as hereinbefore defined.

Intermediates of general formula VIII may be prepared by the halogenation of compounds of general formula XVI shown hereinafter, wherein R and R¹ are as hereinbefore defined, for example by heating with bromine or iodine in the presence of concentrated nitric acid.

The agriculturally acceptable salts may be prepared from the compounds of general formula I by the application or adaptation of known methods.

Compounds of formulae II, III, V, IX, X, XI, XII, XIII, XIV, XV and XVI are known or may be prepared by the application or adaptation of known methods.

Those skilled in the art will appreciate that some compounds of general formula I may be prepared by the interconversion of other compounds of general formula I, and such interconversions constitute yet more features of the present invention. Examples of such interconversions include :-

Compounds in which R, or R² represents a cyano group may be prepared from compounds in which R or R² represents a group -CO₂R³ wherein R³ is other than hydrogen via hydrolysis to the corresponding carboxylic acid, in which R³ is hydrogen, conversion to the corresponding acid halide, for example by treatment with thionyl chloride or oxalyl chloride, treatment with ammonia to give the amide, and dehydration, for example by means of phosphorus oxychloride.

Compounds in which R or R² represents a group -COR⁵ may be prepared from corresponding compounds in which R or R² represents a cyano group by means of a reaction with an organometallic reagent, for example a Grignard reagent of the appropriate structure.

Compounds in which R² represents -SOR⁵ or -SO₂R⁵ may be prepared by oxidation of compounds in which R² represents -SR⁵ using for example m-chloroperbenzoic acid in an inert solvent such as dichloromethane at a temperature from -40°C to 0°C.

Compounds in which R or R² represent -CO₂R³ wherein R³ represents an alkyl group may be converted into other compounds in which R or R² represent -CO₂R³ by transesterification of the ester group, for example by the reaction of a compound in which R or R² represents -CO₂CH₃ with an alkoxide salt of formula M-OR³ (wherein M represents a metal cation e.g. sodium or potassium) wherein R³ is not CH₃, in an alcohol solvent R³OH optionally in the presence of an acid catalyst such as sulphuric acid or boron trifluoride etherate. The reaction is preferably carried out at a temperature from 15°C to the reflux temperature of the mixture. 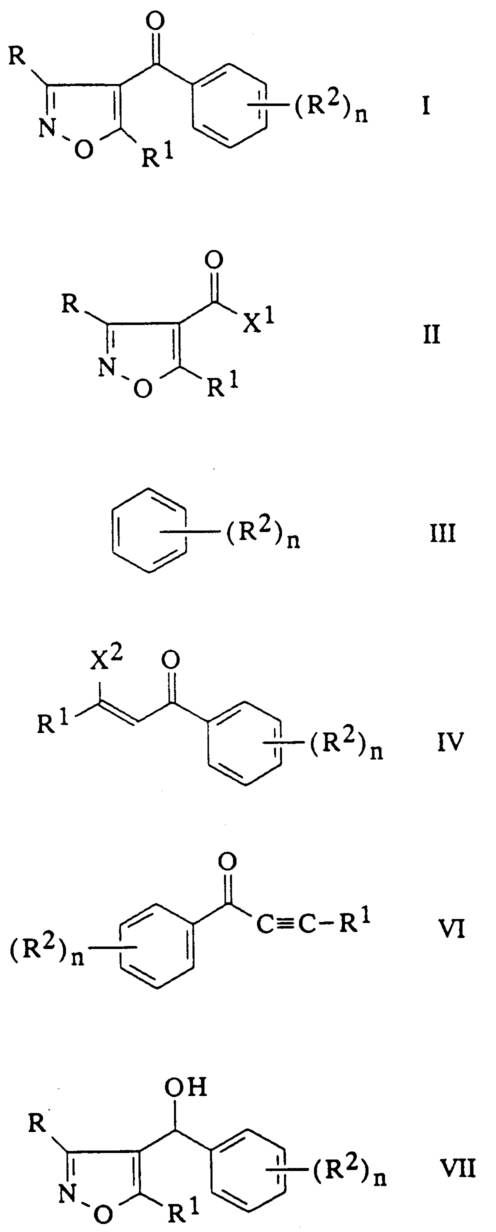 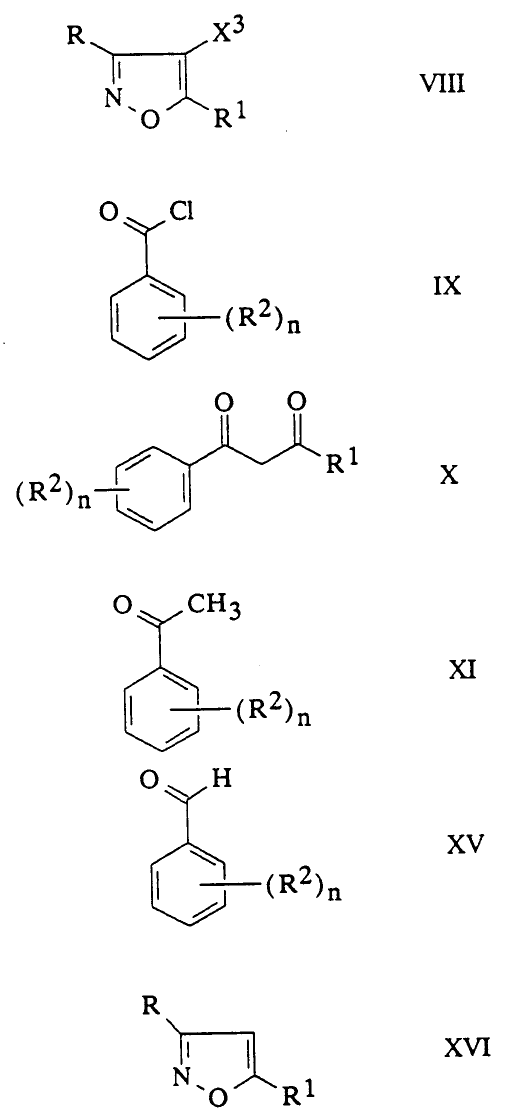 The following Examples illustrate the preparation of compounds of general formula I.

### EXAMPLE 1

A solution of acetohydroximoyl chloride (1.3g) in toluene (25ml) was added progressively to a solution of 3-(N,N-dimethylamino)-1-(2-nitro-4-trifluoromethylphenyl)-prop-2-en-1-one (2.0g) and triethylamine (1.5g) in toluene (25ml). The resulting suspension was stirred for 5 hours, filtered and the filtrate evaporated to dryness. The residue was subjected to chromatography, eluting with a mixture of petroleum spirit and ethyl acetate. The product was recrystallized from cyclohexane to give compound A), (0.66g) in the form of a white solid, m.p. 104°C.

By proceeding in a similar manner the following compounds of general formula I were prepared from the appropriately substituted starting materials:

| **Cmpd. No.** | **R** | **R**^{**1**} | **(R**^{**2**}**)**_{**n**} | **m.p./NMR** |
|---|---|---|---|---|
| B | 1-Methylethyl | H | 2-NO₂-4-CF₃ | 92°C |
| C | CO₂Et | H | 2-NO₂-4-CF₃ | 65°C |
| D | Br | H | 2-NO₂-4-CF₃ | 115°C |
| E | CF₃ | H | 2-NO₂-4-CF₃ | 117°C |
| G | CO₂Et | CH₃ | 2-NO₂-4-CF₃ | 62°C |
| I | CO₂Et | Cyclopropyl | 2-NO₂-4-CF₃ | (CDCl₃) d=1.1(t,3H) 1.2-1.5(m,4H) 2.7(m,1H) 3.9(q.2H) 7,5(d,1H) 7.9(d,1H) 8.4 (s,1H) |
| K | CO₂Et | Cyclopropyl | 2-Cl-4-SO₂Me | 110°C |
| L | CO₂Et | Cyclopropyl | 2-CF₃-4-SO₂Me | 124.5-126°C |
| M | CO₂Me | Cyclopropyl | 2-Cl-4-SO₂Me | 143-144°C |
| O | CO₂Et | Cyclopropyl | 2-SO₂Me | 97-98°C |
| P | CO₂Me | Cyclopropyl | 2-CF₃-4-SO₂Me | 152.5-153°C |
| Q | CO₂Et | Cyclopropyl | 2-NO₂-4-SO₂Me | 131-132°C |
| R | CO₂Et | Cyclopropyl | 2,3-Cl₂-4-SO₂Me | 202-203°C |
| S | CO₂Et | Cyclopropyl | 2-Cl-3-MeO-4-SO₂Me | 130-132°C |
| U | CO₂Et | Cyclopropyl | 2-SO₂Me-4-Cl | 118-119°C |
| V | CO₂Et | Cyclopropyl | 2-NO₂-4-Cl | 83-84°C |
| W | CO₂Et | Cyclopropyl | 2-CF₃-4-Cl | 59-60°C |

### EXAMPLE 2

A solution of n-butyllithium in hexane (2.5M, 20 ml) was added dropwise to a solution of 5-cyclopropyl-4-iodo-3-methylisoxazole (12.4g) in ether (200 ml) , whilst maintaining the temperature below -60°C. The mixture was stirred for 1 hour and a solution of 2-nitro-4-trifluoromethylbenzoyl chloride (12.68 g) in ether (100 ml) was added dropwise whilst maintaining the temperature below -60°C. The mixture was stirred for 1 hour and allowed to warm to 0°C. Hydrochloric acid (2M, 150 ml) was added and the layers were separated. The organic layer was washed with water, saturated sodium metabisulphate solution, water, dried (anhydrous sodium sulphate) and filtered. The filtrate was evaporated to dryness and the residue was triturated with a mixture of petroleum spirit and ether (5:1) and filtered. The solid was recrystallized from cyclohexane to give compound F) (11.3g) as a white solid, m.p. 110°C.

### EXAMPLE 3

A mixture of ethyl 5-methyl-4-(2-nitro-4-trifluoromethylbenzoyl)-isoxazole-3-carboxylate (30g) and concentrated aqueous ammonia (60 ml) was stirred at room temperature overnight. Water (200 ml) was added and the mixture was extracted with ether. The combined extracts were washed with water, dried (anhydrous sodium sulphate) and filtered. The filtrate was evaporated to dryness and the residue was purified by chromatography on silica gel eluted with a mixture of ethyl acetate and petroleum spirit (1:5) to give compound H) (0.44 g) as a white solid, m.p. 144°C.

By proceeding in a similar manner the following compound of general formula I was prepared from the appropriately substituted starting material:

| **Cpd. No.** | **R** | **R**^{**1**} | **(R**^{**2**}**)**_{**n**} | **m.p.** |
|---|---|---|---|---|
| N | CONH₂ | Cyclopropyl | 2-Cl-4-SO₂Me | 202-203°C |

### EXAMPLE 4

A mixture of 5-methyl-4-(2-nitro-4-trifluoromethylbenzoyl)-isoxazole-3-carboxamide (1.2 g) and phosphorus oxychloride (10 ml) was stirred at room temperature for 1.5 hours and at 50-60°C for 1 hour. The mixture was poured into water (50 ml), with cooling as necessary, and extracted with dichloromethane. The combined extracts were washed with water, dried (arhydrous sodium sulphate) and filtered. The filtrate was evaporated to dryness and the residue was purified by chromatography on silica gel eluted with a mixture of ethyl acetate and petroleum spirit (1:5). The product was recrystallized from cyclohexane to give compound J) (0.65g) as an off-white solid, m.p. 104°C.

By proceeding in a similar manner the following compound of general formula I was prepared from the appropriately substituted starting materials:

| **Cmpd. No.** | **R** | **R**^{**1**} | **(R**^{**2**}**)**_{**n**} | **m.p.** |
|---|---|---|---|---|
| T | CN | Cyclopropyl | 2-Cl-4-SO₂Me | 123-123.5°C |

### EXAMPLE 5

A mixture of 3-cyclopropyl-3-diethylamino-1-[2-(methylthio)-4-trifluoromethylphenyl]-prop-2-en-1-one (9.4g), ethyl chloroximidoacetate (6.0g) and 4A molecular sieve (35g) in dichloromethane was stirred for 60 hours. The mixture was filtered and the filtrate was evaporated to dryness and the residue was recrystallized from n-hexane to give compound X) (3.6g) as an off-white solid, m.p. 97-98°C.

By proceeding in a similar manner the following compounds of general formula I were prepared from the appropriately substituted starting materials:

| **Cmpd. No.** | **R** | **R**^{**1**} | **(R**^{**2**}**)**_{**n**} | **m.p./NMR** |
|---|---|---|---|---|
| Y | CO₂Et | Cyclopropyl | 2-CF₃-4-SMe | 97-98°C |
| CC | CO₂Et | Cyclopropyl | 2-F-4-SO₂Me | 116-117°C |
| DD | CO₂Et | Cyclopropyl | 2-Cl-4-SO₂Et | 136-137°C |
| EE | CO₂Et | Cyclopropyl | 2-SO₂Me-4-Br | 93-95°C |
| FF | CO₂Et | Cyclopropyl | 2-Br-4-SO₂Me | 139-140°C |
| GG | CO₂Et | Cyclopropyl | 2-Cl-4-SMe | 75-76°C |
| II | COCH₃ | Cyclopropyl | 2-Cl-4-SO₂Me | 121.5°C |

### EXAMPLE 6

3-Chloroperoxybenzoic acid (1.9g) was added to a solution of ethyl 5-cyclopropyl-4-[2-(methylthio)-4-trifluoromethylbenzoyl]-isoxazole-3-carboxylate (2.7g) in dichloromethane whilst maintaining the temperature below -10°C. The mixture was stirred at -5°C for 1.5 hours and allowed to warm to room temperature. A solution of sodium metabisulphite in water was added and the layers were separated. The organic layer was washed with water, dried (anhydrous sodium sulphate) and filtered. The filtrate was evaporated to dryness and the residue was purified by chromatography on silica eluted with a mixture of ethyl acetate and hexane (1:3) to give compound Z) (1.2g) as a white solid, m.p. 140-141°C.

By proceeding in a similar manner the following compounds of general formula I were prepared from the appropriately substituted starting materials.

| **Cmpd. No.** | **R** | **R**^{**1**} | **(R**^{**2**}**)**_{**n**} | **m.p./NMR** |
|---|---|---|---|---|
| AA | CO₂Et | Cyclopropyl | 2-CF₃-4-SOMe | 65-66°C |
| BB | CO₂Et | Cyclopropyl | 2-SO₂Me-4-CF₃ | 101-102°C |
| HH | CO₂Et | Cyclopropyl | 2-Cl-4-SOMe | 100-101°C |
| NN | CO₂Me | Cyclopropyl | 2-SOMe-4-Cl | 82-83°C |

### EXAMPLE 7

Sodium hydride (0.15g) was added to isopropanol. This was followed by the addition of methyl 4-(2-chloro-4-methylsulphonylbenzoyl)-5-cyclopropylisoxazole-3-carboxylate (1.9g). The mixture was stirred and heated at reflux for 2.5 hours, cooled and water was added. It was extracted with ethyl acetate and the organic layer was washed with water, dried (anhydrous sodium sulphate) and filtered. The filtrate was evaporated to dryness. The residue was triturated with ether and filtered. The solid was treated with ethyl acetate and filtered. The filtrate was passed through a short column of silica and the eluate was evaporated to dryness to give compound JJ) (0.25g) as a white solid, m.p. 149-150°C.

### EXAMPLE 8

A mixture of magnesium (0.35g) and carbon tetrachloride (0.5ml) in methanol was warmed until all of the magnesium had dissolved. 1-(4-chloro-2-methylsulphonylphenyl)-3-cyclopropylpropane-1,3-dione (4.0g) was added and the mixture was stirred and heated at reflux for 2 hours. It was then cooled and evaporated to dryness. The residue was dissolved in dichloromethane and a solution of methyl chloro-oximidoacetate (2.75g) in dichloromethane was added. The mixture was stirred at room temperature overnight, washed with hydrochloric acid (2M), water, dried (anhydrous sodium sulphate) and filtered. The filtrate was evaporated to dryness and the residue was triturated with ether and filtered. The solid was recrystallized from ethanol to give compound KK) (1.7g) as a pale orange solid, m.p. 146-147°C.

By proceeding in a similar manner the following compounds of general formula I were prepared from the appropriately substituted starting materials:

| **Cmpd. No.** | **R** | **R**^{**1**} | **(R**^{**2**}**)**_{**n**} | **m.p./NMR** |
|---|---|---|---|---|
| LL | CO₂Me | Cyclopropyl | 2-SO₂-Me-4-CF₃ | 130-131°C |
| MM | CO₂Me | Cyclopropyl | 2-SMe-4-Cl | 112-113°C |

The following Examples illustrate the preparation of intermediates relating to the present invention:-

### EXAMPLE 1(a)

A mixture of 2-nitro-4-trifluoromethyl-acetophenone (10g) and dimethylformamide dimethylacetal (20 ml) was heated at reflux for 2 hours, then evaporated to dryness and the residue was mixed with cyclohexane (20 ml), filtered to give 3-(N,N-dimethylamino)-1-(2-nitro-4-trifluoromethylphenyl)prop-2-en-1-one, (12.1g), in the form of a red solid, m.p. 107°C.

### EXAMPLE 2(a)

A mixture of diethyl 2-nitro-4-trifluoromethylbenzoylmalonate (74.2g), concentrated sulphuric acid (30 ml), water (170 ml) and glacial acetic acid (250ml) was heated at reflux for 2 hours, then cooled, basified with aqueous sodium hydroxide solution (2M) and extracted with diethyl ether. The organic phase was washed with water, dried over anhydrous sodium sulphate, and filtered. The filtrate was evaporated to dryness and the resulting residue was recrystallized from a mixture of diethyl ether and n-hexane (1:10 v/v), to give 2-nitro-4-trifluoromethylacetophenone (30.4g), in the form of an off-white solid, m.p. 65°C.

### EXAMPLE 3(a)

A mixture of magnesium turnings (4.94g) and carbon tetrachloride (2ml) in ethanol (35 ml) was warmed to 50°C until the reaction started. Diethyl ether (150 ml) was then added cautiously, with stirring. A solution of diethyl malonate (32.5 g) in diethyl ether (50 ml) was then added dropwise, then stirred and heated at reflux for 2 hours. After cooling the mixture, diethyl ether (150 ml) was added. A solution of 2-nitro-4-trifluoromethylbenzoyl chloride (50.7g) in diethyl ether (100 ml) was added dropwise. The mixture was heated at reflux for 2 hours. After cooling, hydrochloric acid (2M,200ml) was added and the layers were separated. The organic layer was washed with water, dried (anhydrous sodium sulphate), and filtered. The filtrate was evaporated to dryness, to give diethyl 2-nitro-4-trifluoromethylbenzoylmalonate (72.6g), in the form of an off-white solid, m.p. 60°C.

### EXAMPLE 4(a)

A mixture of 2-nitro-4-trifluoromethylbenzoyl chloride (8.0g) and triphenylphosphine (16.5g) in dry acetone (75 ml) was treated with bis-(triphenylphosphine)tetrahydroboratocopper(I) (19.8g). The resulting suspension was stirred for 1 hour, filtered and washed with diethyl ether. The filtrate was evaporated to dryness and the resulting residue was subjected to chromatography, eluting with a mixture of diethyl ether and n-hexane (1:5 v/v), to give 2-nitro-4-trifluoromethylbenzaldehyde (5.91g) in the form of an off-white solid, m.p. 63°C.

### EXAMPLE 5(a)

Concentrated nitric acid (10 ml) was added dropwise to a stirred heated mixture of iodine (23.8g) and 5-cyclopropyl-3-methylisoxazole (24.6g containing approximately 20 % 3-cyclopropyl-5-methylisoxazole) whilst maintaining the temperature in the range 90-95°C. The mixture was stirred and heated at 95-100°C for 1 hour. It was cooled, poured into cold water and extracted with ether. The combined extracts were washed with aqueous sodium bicarbonate solution (saturated ), aqueous sodium metabisulphate solution (saturated ), water, dried over anhydrous sodium sulphate and filtered. The filtrate was evaporated to dryness and the residue was triturated with petroleum spirit and filtered. The solid was recrystallized from petroleum spirit to give 5-cyclopropyl-4-iodo-3-methylisoxazole (25.6g) as a white solid, m.p. 71°C.

### EXAMPLE 6(a)

A mixture of 1-cyclopropylbutan-1,3-dione (65.3g), hydroxylamine hydrochloride (36.6g) and anhydrous potassium carbonate (71.8 g) in ethanol (375 ml) was stirred and heated at reflux for 2 hours. The mixture was cooled and filtered and the filtrate was evaporated to dryness. The residue was distilled under reduced pressure to give 5-cyclopropyl-3-methyl isoxazole containing approximately 20 % 3-cyclopropyl-5-methylisoxazole (51.85g) as a clear oil, b.p. 74°C / 1.6 kPa (12 mm.Hg).

### EXAMPLE 7(a)

A solution of 2-nitro-4-trifluoromethylacetophenone (20.7g) and dimethylacetamide dimethyl acetal (13.2g) in toluene (100 ml) was stirred and heated at reflux overnight. Further dimethylacetamide dimethyl acetal (13.2g) was added and the mixture was stirred and heated at reflux for 1.5 hours . The mixture was cooled, washed with water, dried (anhydrous sodium sulphate) and filtered. The filtrate was evaporated to dryness and the residue was triturated with a mixture of ether and cyclohexane (1:20) and filtered. The yellow solid was recrystallized from cyclohexane to give 3-dimethylamino-1-(2-nitro-4-trifluoromethylphenyl)-but-2-en-1-one (13.95g) as a yellow solid, m.p. 122°C.

### EXAMPLE 8(a)

2-nitro-4-trifluoromethylbenzoyl chloride (9.4g) was added dropwise to a cooled mixture of 1-cyclopropyl-1-diethylaminoethene (5.1g) and triethylamine (3.7g) in toluene (100 ml) whilst maintaining the temperature below 10°C. The mixture was then stirred at room temperature for 1 hour . The suspension was treated with decolourising charcoal and filtered. The filtrate was evaporated and the residue was purified by chromatography on silica gel eluted with a mixture of ethyl acetate and dichloromethane (1:9) to give 3-cyclopropyl-3-diethylamino-1-(2-nitro-4-trifluoromethylphenyl)-prop-2-en-1-one (4.1g) as a red oil with its ¹H NMR (in CDCl₃) giving peaks at d = 0.7-1.8(m, 11H), 3.5(q, 4H), 5.0(s, 1H), 7.5(d, 1H), 7.7(d, 1H),and 7.95(s, 1H).

By proceeding in a similar manner the following compounds were prepared from the appropriately substituted starting materials:

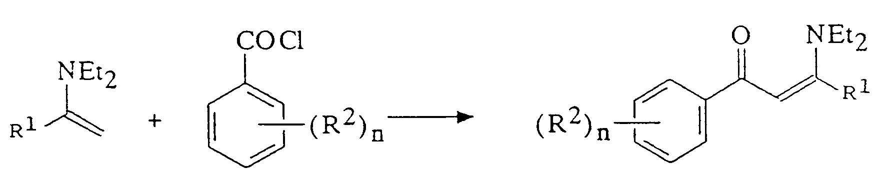

| **R**^{**1**} | **(R**^{**2**}**)**_{**n**} | **NMR** |
|---|---|---|
| Cyclopropyl | 2-Cl-4-SO₂Me | (CDCl₃): 0.6-1.8(m,11H) 3.0(s,3H) 3.5(q,4H) 5.05(s,1H) 7.4 (2,1H) 7.65(d,1H) 7.75(s,1H). |
| Cyclopropyl | 2-CF₃-4-SO₂Me | (CDCl₃): 0.5-1.8(m,11H) 3.1(s,3H) 3.5(q,4H) 5.0(s,1H) 7.5(d,1H) 7.95(d,1H)8.05(s,1H). |
| Cyclopropyl | 2-SO₂Me | (CDCl₃): 0.7-1.8(m,11H) 3.4(s,3H) 3.5(q,4H) 5.1(s,1H) 7.2-7.5(m,3H) 7.75-8.0 (m,1H). |
| Cyclopropyl | 2-NO₂-4-SO₂Me | Crude red oil not further purified. |

| **R**^{**1**} | **(R**^{**2**}**)**_{**n**} | **NMR** |
|---|---|---|
| Cyclopropyl | 2,3-Cl₂-4-SO₂Me | (CDCl₃): 0.5-1.8(m,11H) 3.25(s,3H) 3.55(q,4H) 5.0(s,1H) 7.3(d,1H) 7.9(d,1H). |
| Cyclopropyl | 2-Cl-3-OMe-4-SO₂Me | (CDCl₃): 0.6-1.8(m,11H) 3.2(s,3H) 3.5(q,4H) 4.0(s,3H) 5.0(s,1H) 7.1(d,1H) 7.65(d,1H). |
| Cyclopropyl | 2-SO₂Me-4-Cl | Crude red oil not further purified. |
| Cyclopropyl | 2-CF₃-4-Cl | (CDCl₃): 0.5-1.7(m,11H) 3.45(q,4H) 5.0(s,1H) 7.3(s,2H) 7.4(S,1H). |
| Cyclopropyl | 2-SMe-4-CF₃ | Crude orange oil not further purified. |
| Cyclopropyl | 2-CF₃-4-SMe | Crude orange oil not further purified. |
| Cyclopropyl | 2-F-4-SO₂Me | Crude red oil not further purified. |
| Cyclopropyl | 2-SO₂Me-4-Br | Crude red oil not further purified. |
| Cyclopropyl | 2-Cl-4-SO₂Et | Crude red oil not further purified. |
| Cyclopropyl | 2-Br-4-SO₂Me | (CDCl₃): 0.6-1.9(m,11H) 3.05(s,3H) 3.55(q,4H) 5.05(s,1H) 7.4(d,1H) 7.7(d,1H) 7.95(s,1H). |
| Cyclopropyl | 2-Cl-4-SMe | (CDCl₃): 0.5-1.8(m,11H) 2.45(s,3H) 3.45(q,4H) 5.15(s,1H) 6.9(d,1H) 7.0(s,1H) 7.25(d,1H). |

### EXAMPLE 9(a)

A solution of titanium tetrachloride (10.4g) in n-hexane (30 ml) was added dropwise with stirring to a solution of cyclopropyl methyl ketone (8.4g) and diethylamine (43.9g) in n-hexane (100 ml) whilst maintaining the temperature below 25°C. The mixture was stirred for 5 hours, filtered and the filtrate was evaporated to dryness to give 1-cyclopropyl-1-diethyl aminoethene (5.3g) as a pale yellow oil, with its ¹H NMR (CDCl₃) giving peaks at d = 0.5-0.8(m, 4H), 1.1(t, 6H), 1.4(m, 1H), 3.2(q, 4H), 3.45(s, 1H), 3.5(s, 1H).

### EXAMPLE 10(a)

A mixture of magnesium (1.5g) and carbon tetrachloride (0.5ml) in methanol was stirred the magnesium had completely dissolved. t-Butyl 3-cyclopropyl-3-oxopropionate (11.3g) was added and the mixture was stirred and heated at reflux for 2 hours. The mixture was evaporated to dryness and the residue was dissolved in toluene. 4-Chloro-2-methylsulphonylbenzoyl chloride was added and the mixture was stirred at room temperature overnight. It was washed with hydrochloric acid and dried by azeotropic removal of water. 4-Toluenesulphonic acid (0.5g) was added and the mixture was heated at reflux for 5 hours. The cooled solution was washed with water, dried (anhydrous sodium sulphate) and filtered. The filtrate was evaporated to dryness to give 1-(4-chloro-2-methylsulphonylphenyl)-3-cyclopropylpropan-1,3-dione (16.2g) as an orange oil, NMR (CDCl₃) 0.8-1.2(m,4H) 1.5-1.9(m,1H) 3.3(s,3H) 5.8(s,1H) 7.3-7.6(m,2H) 7.9(s,1H).

By proceeding in a similar manner the following compounds were prepared from the appropriately substituted starting materials.

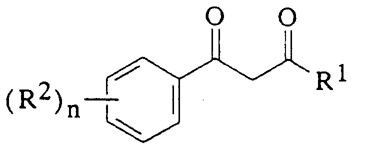

| **R**^{**1**} | **(R**^{**2**}**)**_{**n**} | **NMR** |
|---|---|---|
| Cyclopropyl | 2-SO₂Me-4-CF₃ | (CDCl₃) 0.8-1.4(m,4H) 1.5-1.8(m,1H) 3.3(s,3H) 5.85(s,2H) 7.5 (d,1H) 7.8(d,1H) 8.2(s,1H) |

| **R**^{**1**} | **(R**^{**2**}**)**_{**n**} | **NMR** |
|---|---|---|
| Cyclopropyl | 2-SMe-4-Cl | (CDCl₃) 0.7-1.35(m,4H) 1.4-1.9(m,1H) 2.4(s,3H) 6.1(s,2H) 7.0-7.9(m,3H) |

Benzoyl chlorides were prepared by heating the appropriately substituted benzoic acids at reflux with thionyl chloride for 3 hours. The excess thionyl chloride was removed by evaporation and the benzoyl chlorides were used directly without further purification.

### EXAMPLE 11(a)

Potassium permanganate (316g) was added with stirring to a suspension of 4-bromo-2-methylsulphenyltoluene (90.5g) in water whilst maintaining the mixture at reflux. The mixture was stirred and heated at reflux for 3 hours. The mixture was filtered and the residue was washed with hot water. The filtrate was cooled to room temperature and extracted with ethyl acetate. The aqueous solution was acidified to pH 1, saturated with sodium chloride and extracted with ethyl acetate. The organic layer was washed with water, dried (anhydrous magnesium sulphate) and filtered. The filtrate was evaporated to dryness to give 4-bromo-2-acid (44.6g) as a light brown solid, m.p. 220-220.5°C.

By proceeding in a similar manner the following compounds were prepared from the appropriately substituted starting materials:
4-chloro-2-methylsulphonylbenzoic acid; NMR (CDCl₃ + DMSO-d₆): 3.35 (s,3H), 7.5-7.8(m,2H), 7.9 (s,1H), 8.2-8.6 (bs,1H);
2-fluoro-4-methylsulphonylbenzoic acid; m.p.187-189°C.

### EXAMPLE 12(a)

Hydrogen peroxide (30%) was added to a cooled solution of 2-methylsulphenyl-4-trifluoromethylbenzoic acid (6.0g) and acetic anhydride(3.6ml) in acetic acid at 10°C. The mixture was allowed to warm slowly to room temperature and stirred for 0.5 hours. It was stirred and heated at 65°C for 3 hours. After cooling the mixture was poured into ice and extracted with ether. The organic layer was washed with water, aqueous ferrous sulphate solution, dried (anhydrous magnesium sulphate) and filtered. The filtrate was evaporated to dryness to give 2-methylsulphonyl-4-trifluoromethylbenzoic acid (5.54g) as a white solid, m.p. 155.5-156.5°C.

### EXAMPLE 13(a)

n-Butyllithium (2.5M solution in hexane; 25 ml) was added under an inert atmosphere to a stirred solution 4-bromo-3-methylsulphenyl-benzotrifluoride (16.4g) in ether whilst maintaining the temperature below-70°C. The mixture was stirred at -70°C for 2 hours and then poured onto solid carbon dioxide pellets. The mixture was stirred for 10 minutes and aqueous hydrochloric acid was added. The layers were separated and the aqueous layer was extracted with ether. The combined organic layers were washed with water, dried (anhydrous magnesium sulphate) and filtered.

The filtrate was evaporated and the residue was triturated with cyclohexane and filtered to give 2-methylsulphenyl-4-trifluoromethylbenzoic acid (12.4g) as a white solid, NMR [(CDCl₃) + DMSO-d₆]: 2.45 (s,3H), 7.2(d,1H), 7.3(s,1H), 8.0 (d,1H), 10.7-11.1 (bs,1H).

### EXAMPLE 14(a)

t-Butyl nitrite (4ml) was added to a mixture of 5-chloro-2-methylaniline (4g) and dimethyl disulphide (26.3g) in chloroform. After the reaction started t-butyl nitrite (17.7ml) and 5-chloro-2-methylaniline (16g) were added simultaneously. The mixture was stirred at room temperature for 2 hours and left to stand overnight. The mixture was washed with water, aqueous hydrochloric acid (2M), water, dried (anhydrous magnesium sulphate) and filtered. The filtrate was evaporated to dryness to give 4-chloro-2-methylsulphenyltoluene (24.6g) as a red oil, NMR (CDCl₃): 2.13 (s,3H), 2.2 (s,3H), 6.85 (s,2H), 7.0 (s,1H).

By proceeding in a similar manner the following compounds were prepared from the appropriately substituted starting materials:
4-bromo-3-methylsulphenylbenzotrifluoride, b.p. 84-88°C at 2.7 hPa (2 mm) Hg;
2-fluoro-4-methylsulphenyltoluene, NMR (CDCl₃): 2.2 (s,3H), 2.45 (s,3H), 6.6-7.1 (m,3H).

### EXAMPLE 15(a)

A cooled solution of sodium nitrite (5.8g) in concentrated sulphuric acid (50 ml) was added dropwise to a stirred solution of 4-methyl-3-methylsulphenylaniline (12.8g) in glacial acetic acid at 20°C. The resulting suspension was added to a mixture of copper (I) bromide (12g), aqueous hydrobromic acid (48-50%) and ice. The mixture was stirred at room temperature for 3 hours then diluted with water and extracted with ethyl acetate. The organic layer was washed with water, aqueous sodium hydroxide (2M), dried (anhydrous magnesium sulphate) and filtered. The filtrate was evaporated to dryness. The residue was triturated with hot cyclohexane and filtered. The filtrate was evaporated to dryness to give 4-bromo-2-methylsulphenyltoluene (8.6g) as a brown oil, NMR (CDCl₃): 2.15(s,3H), 2.2(s,3H), 6.5-7.1 (m,3H).

### EXAMPLE 16(a)

Concentrated hydrochloric acid (128 ml) was added slowly to a suspension of 2-methylsulphenyl-4-nitrotoluene (36.6g) in methanol. Iron powder (36g) was added with stirring whilst maintaining the temperature below 50°C. The mixture was stirred at room temperature for 4 hours. The mixture was poured into water neutralized (by the addition of sodium carbonate), filtered and the residue was extracted with dichloromethane. The aqueous layer was extracted with dichloromethane and the combined organic layers were washed with aqueous sodium chloride solution, dried (anhydrous magnesium sulphate) and filtered. The filtrate was evaporated to dryness and the residue was purified by column chromatography on silica eluted with a mixture of ethyl acetate and n-hexane to give 4-methyl-3-methylsulphenylaniline (12.8g) as an orange solid, NMR (CDCl₃) 2.2(s,3H), 2.35 (s,3H), 3.45 (s,2H), 6.1-6.9 (m,3H).

### EXAMPLE 17(a)

A solution of 4-chloro-2-trifluoromethyliodobenzene (100g) in ether was added to a suspension of magnesium (8g) in ether at a such a rate as to maintain the mixture at reflux. The mixture was stirred at reflux for 1 hour and then cooled to 0°C. Carbon dioxide was then passed into the stirred mixture for 3 hours. Hydrochloric acid (2M) was added and the resulting layers separated. The organic layer was washed with water and then extracted into aqueous sodium carbonate solution and this aqueous extract was acidified to pH 1 and extracted with ether. The ether layer was washed with saturated sodium chloride solution, dried (anhydrous magnesium sulphate) and the solvent evaporated to give 4-chloro-2-trifluoromethylbenzoic acid (57.1g) as a brown solid, m.p. 106.5-108°C.

According to a feature of the present invention, there is provided a method for controlling the growth of weeds (ie. undesired vegetation) at a locus which comprises applying to the locus a herbicidally effective amount of at least one isoxazole derivative of general formula I. For this purpose, the isoxazole derivatives are normally used in the form of herbicidal compositions (ie. in association with compatible diluents or carriers and/or surface active agents suitable for use in herbicidal compositions), for example as hereinafter described, and those compositions are also part of the invention.

The compounds of general formula I show herbicidal activity against dicotyledonous (ie. broad-leafed) and monocotyledonous (eg. grass) weeds by pre- and/or, post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula I may be used to control the growth of
broad-leafed weeds, for example, Abutilon theophrasti, Amaranthus retroflexus, Bidens pilosa, Chenopodium album, Galium aparine, Ipomoea spp, eg. Ipomoea purpurea, Sesbania exaltata, Sinapis arvensis, Solanum nigrum, Viola arvensis and Xanthium strumarium, and
grass weeds, for example, Alopecurus myosuroides, Digitaria sanguinalis, Echinochloa crus-galli, Eleusine indica and Setaria spp, eg. Setaria faberii and Setaria viridis, and Sorghum halepense, and
sedges, for example, Cyperus esculentus, Cyperus iria, Cyperus rotundus and Eleocharis acicularis.

The amounts of compounds of general formula I applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.01kg and 5kg of active material per hectare give good results. However, it is to be understood that higher or lower application rates may be used, depending upon the particular problem of weed control encountered.

The compounds of general formula I may be used to control selectively the growth of weeds, for example to control the growth of those species hereinbefore mentioned, by pre- or post-emergence application in a directional or non-directional fashion, eg. by directional or non-directional spraying, to a locus of weed infestation which is an area used, or to be used, for growing crops, for example cereals, eg. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, eg. the crops hereinbefore mentioned, application rates between 0.01kg and 4.0kg, and preferably between 0.01kg and 2.0kg, of active material per hectare are particularly suitable.

The compounds of general formula I may also be used to control the growth of weeds, especially those indicated above, by pre- or post-emergence application in established orchards and other tree-growing areas, for example forests, woods and parks, and plantations, eg. sugar cane, oil palm and rubber plantations. For this purpose they may be applied in a directional or non-directional fashion (eg. by directional or non-directional spraying) to the weeds or to the soil in which they are expected to appear, before or after planting of the trees or plantations at application rates between 0.25kg and 5.0kg, and preferably between 0.5kg and 4.0kg of active material per hectare.

The compounds of general formula I may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (eg. by directional or non-directional spraying) at application rates between 1.0kg and 20.0kg, and preferably between 5.0 and 10.0kg, of active material per hectare are particularly suitable for this purpose.

When used to control the growth of weeds by pre-emergence application, the compounds of general formula I may be incorporated into the soil in which the weeds are expected to emerge. It will be appreciated that when the compounds of general formula I are used to control the growth of weeds by post-emergence application, ie. by application to the aerial or exposed portions of emerged weeds, the compounds of general formula I will also normally come into contact with the soil and may also then exercise a pre-emergence control on later-germinating weeds in the soil.

Where especially prolonged weed control is required, the application of the compounds of general formula I may be repeated if required.

According to a further feature of the present invention, there are provided compositions suitable for herbicidal use comprising one or more of the isoxazole derivatives of general formula I in association with, and preferably homogeneously dispersed in, one or more compatible herbicidally- acceptable diluents or carriers and/or surface active agents [ie. diluents or carriers and/or surface active agents of the type generally accepted in the art as being suitable for use in herbicidal compositions and which are compatible with compounds of general formula I]. The term "homogeneously dispersed" is used to include compositions in which the compounds of general formula I are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula I.

The herbicidal compositions may contain both a diluent or carrier and surface-active (eg. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, eg. nonyl-or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10%, eg. from 0.05% to 10%, of surface-active agent but, if desired, herbicidal compositions according to the present invention may comprise higher proportions of surface-active agent, for example up to 15% in liquid emulsifiable suspension concentrates and up to 25% in liquid water soluble concentrates.

Examples of suitable solid diluents or carriers are aluminium silicate, talc, calcined magnesia, kieselguhr, tricalcium phosphate, powdered cork, adsorbent carbon black and clays such as kaolin and betonite. The solid compositions (which may take the form of dusts, granules or wettable powders) are preferably prepared by grinding the compounds of general formula I with solid diluents or by impregnating the solid diluents or carriers with solutions of the compounds of general formula I in volatile solvents, evaporating the solvents and, if necessary, grinding the products so as to obtain powders. Granular formulations may be prepared by absorbing the compounds of general formula I (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of general formula I may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Preferred herbicidal compositions according to the present invention are aqueous suspension concentrates which comprise from 10 to 70% of one or more compounds of general formula I, from 2 to 10% of surface-active agent, from 0.1 to 5% of thickener and from 15 to 87.9% of water; wettable powders which comprise from 10 to 90% of one or more compounds of general formula I, from 2 to 10% of surface-active agent and from 8 to 88% of solid diluent or carrier; soluble powders which comprise from 10 to 90% of one or more compounds of general formula I, from 2 to 40% of sodium carbonate and from 0 to 88% of solid diluent; liquid water soluble concentrates which comprise from 5 to 50%, eg. 10 to 30%, of one or more compounds of general formula I, from 5 to 25% of surface-active agent and from 25 to 90%, eg. 45 to 85%, of water miscible solvent, eg. dimethylformamide, or a mixture of water-miscible solvent and water; liquid emulsifiable suspension concentrates which comprise from 10 to 70% of one or more compounds of general formula I, from 5 to 15% of surface-active agent, from 0.1 to 5% of thickener and from 10 to 84.9% of organic solvent; granules which comprise from 1 to 90%, eg. 2 to 10% of one or more compounds of general formula I, from 0.5 to 7%, eg. 0.5 to 2%, of surface-active agent and from 3 to 98.5%, eg. 88 to 97.5%, of granular carrier and emulsifiable concentrates which comprise 0.05 to 90%, and preferably from 1 to 60% of one or more compounds of general formula I, from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula I in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled for example alachlor [2-chloro-2′,6′-diethyl-N-(methoxy-methyl)-acetanilide], atrazine [2-chloro-4-ethylamino-6-isopropylamino-1,3,5-triazine], bromoxynil [3,5-dibromo-4-hydroxybenzonitrile], chlortoluron [N′- (3-chloro-4-methylphenyl)-N,N-dimethylurea], cyanazine [2-chloro-4-(1-cyano-1-methylethylamino)-6-ethylamino-1,3,5-triazine], 2,4-D [2,4-dichlorophenoxy- acetic acid], dicamba [3,6-dichloro-2-methoxybenzoic acid], difenzoquat [1,2-dimethyl-3,5-diphenyl-pyrazolium salts], flampropmethyl [methyl(+)-2-(N- benzoyl-3-chloro-4-fluoroanilino)-propionate], fluometuron [N′-(3-trifluoromethylphenyl)-N,N-dimethylurea], isoproturon [N′-(4-isopropylphenyl)-N,N- dimethylurea], insecticides, eg. synthetic pyrethroids, eg. permethrin and cypermethrin, and fungicides, eg. carbamates eg. methyl N-(1-butyl-carbamoyl- benzimidazol-2-yl)carbamate, and triazoles eg. 1-(4-chloro-phenoxy)-3,3-dimethyl-1-(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the isoxazole derivatives of general formula I or, as is preferred, a herbicidal composition as hereinbefore described, and preferably a herbicidal concentrate which must be diluted before use, comprising at least one of the isoxazole derivatives of general formula I within a container for the aforesaid derivative or derivatives of general formula I, or a said herbicidal composition, and instructions physically associated with the aforesaid container setting out the manner in which the aforesaid derivative or derivatives of general formula I or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the isoxazole derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01kg and 20kg of active material per hectare in the manner and for the purposes hereinbefore described.

The following Examples illustrate herbicidal compositions according to the present invention:

### EXAMPLE C1

A wettable powder was formed from:
* active ingredient (compound A): 50% w/w
* nonylphenol/ethylene oxide condensate containing 9 moles of ethylene oxide per mol of phenol: 5% w/w
* silicon dioxide of microfine particle size: 5% w/w
* synthetic magnesium silicate carrier: 40% w/w
by absorbing the condensate on the silicon dioxide, mixing with the other ingredients and grinding the mixture in a hammer mill to give a wettable powder.

Similar wettable powders may be prepared as described above by replacing the isoxazole (compound A) by other compounds of general formula I.

### EXAMPLE C2

An aqueous suspension concentrate was formed from:
* active ingredient (compound A): 50% w/v
* nonylphenol/ethylene oxide condensate containing 9 moles of ethylene oxide per mol of phenol: 1 % w/v
* sodium salt of polycarboxylic acid: 0.2% w/v
* Ethylene glycol : 5 % w/v
* polysaccharide xanthan gum thickener: 0.15 % w/v
* water to 100% by volume
by intimately mixing the ingredients and grinding in a ball-mill for 24 hours.

Similar aqueous concentrates may be prepared as described above by replacing the isoxazole (compound A) by other compounds of general formula I.

Representative compounds of general formula I have been used in herbicidal applications or use according to the following procedures.

### Method of used of herbicidal compounds:

Appropriate quantities of the compounds used to treat the plant were dissolved in acetone to give solutions equivalent to an application rate of up to 4000g of test compounds per hectare (g/ha). These solutions were applied at 260 litres of spray fluid per hectare.

### a) Pre-emergence weed control

Seeds (weeds or crops) were sown in loam soil pots.

The compounds of the invention were applied to the soil surface as described above.

### b) Post-emergence weed control

Weed species were grown until ready for spraying with the compounds used to treat the plants. The growth stages of the plant at spraying were as follows:

| 1) Broad-leafed weeds: | |
|---|---|
| Abutilon theophrasti: | 1-2 leaves. |
| Amaranthus retroflexus: | 1-2 leaves. |
| Galium aparine: | 1-2 whorls. |
| Sinapsis arvensis: | 2 leaves. |
| Ipomoea purpurea: | 1-2 leaves. |
| Xanthium strumarium: | 2 leaves. |

| 2) Grass weeds: | |
|---|---|
| Alopecurus myosuroides: | 2 leaves. |
| Avena fatua: | 1-2 leaves. |
| Echinochloa crus-galli | 2-3 leaves. |
| Setaria viridis: | 2-3 leaves. |

| 3) Sedges: | |
|---|---|
| Cyperus esculentus: | 3 leaves. |

### c) Crop tolerance

Compounds of the invention were applied pre-emergence as in (a) or post emergence (3-leaf stage) to crops as described above.

A single pot of each plant species was allocated to each treatment with unsprayed controls and controls sprayed with acetone alone.

After treatment, the pots were kept in the greenhouse and were watered overhead.

Visual assessment of phytotoxicity was made 17-20 days after spraying. Weed control results were expressed as the percentage reduction in growth or kill of the weeds, in comparison with the plants in the control pots. Crop tolerance was expressed as the percentage damage to crop.

The compounds of the invention, when used at 4kg/ha or less, have shown an excellent level of herbicidal activity together with crop tolerance on the weeds used in the foregoing experiments.

When applied pre-emergence at 4000g/ha compounds B, D, E and F gave at least 70% reduction in growth of one or more of the weed species.

When applied post-emergence at 4000g/ha compounds A, C, D, E and F gave at least 90% reduction in growth of one or more of the weed species.

When applied pre-emergence at 1000g/ha compounds G, H, I, J, K, L, M, N, O, P, Q, R, S, T, U, V, W, X, Y, Z, AA, BB, CC, DD, EE, FF, GG, HH, II, JJ, KK, LL, MM and NN gave at least 90% reduction in growth of one or more of the weed species.

When applied post-emergence at 1000g/ha compounds G, I, K, L, M, N, O, P, Q, R, S, T, U, V, W, X, Y, Z, AA, BB, CC, DD, EE, FF, GG, HH, II, JJ, KK, LL, MM and NN gave at least 90% reduction in growth of one or more of the weed species.

Crop tolerance was observed on at least one of the cereal crops either pre-or post-emergence with less than 30% damage showing that the compounds had good potential for selective control in cereal crops.

## Claims (Claims for the following Contracting State(s): AT, BE, CH, LI, DE, DK, FR, GB, GR, IT, LU, NL, SE)

1. A 4-Benzoyl isoxazole derivative of formula I: 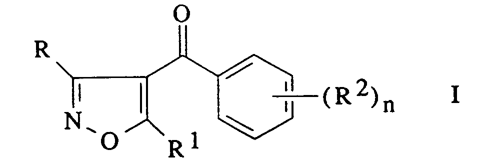 wherein:
R represents :-
a straight- or branched-chain alkyl, alkenyl or alkynyl group containing up to 6 carbon atoms which is optionally substituted by one or more halogen atoms; or
a cycloalkyl group containing from 3 to 6 carbon atoms optionally substituted by one or more substituents selected from groups R⁵, one or more halogen atoms and a group -CO₂R³; or
a group selected from -CO₂R³, -COR⁵, cyano, nitro, -CONR³¹R⁴,
or a halogen atom;
R¹ represents :-
a hydrogen atom, or
a straight- or branched-chain alkyl, alkenyl or alkynyl group containing up to 6 carbon atoms which is optionally substituted by one or more halogen atoms; or
a cycloalkyl group containing from 3 to 6 carbon atoms optionally substituted by one or more substituents selected from groups R⁵ and one or more halogen atoms;
R² represents :-
a halogen atom, or
a group selected from R⁵, -SR⁵, -SOR⁵, -SO₂R⁵, -SO₂NR³¹R⁴, -CO₂R³, -COR⁵, -CONR³¹R⁴, -CSNR³¹R⁴, -OR⁵, a nitro group, a cyano group, a group -O(CH₂)_{q}-OR⁵, or a straight- or branched- chain alkyl group containing up to 6 carbons and which is substituted by OR⁵;
R³, R³¹ and R⁴, which may be the same or different, each represents :-
a hydrogen atom, or
a straight- or branched-chain alkyl group containing up to 6 carbon atoms which is optionally substituted by one or more halogen atoms;
R⁵ represents :-
a straight- or branched-chain alkyl group containing up to 6 carbon atoms which is optionally substituted by one or more halogen atoms;
n represents an integer from 1 to 5;
q represents an integer from 1 to 3;
provided that R and R¹ do not simultaneously represent a methyl group;
or an agriculturally acceptable salt thereof.

2. A compound according to claim 1 in which at least one R² occupies an ortho position adjacent to the carbonyl group linking the phenyl and isoxazolyl rings.

3. A compound according to claim 1 or 2 in which R represents :-
a straight- or branched- chain alkyl group containing up to 6 carbon atoms which is optionally substituted by one or more halogen atoms, or
a cycloalkyl group containing from 3 to 6 carbon atoms, or
a group selected from -COR⁵, -CO₂R³, or
a halogen atom.

4. A compound according to claim 1, 2 or 3 in which R¹ represents :-
a straight- or branched- chain alkyl group containing up to 6 carbon atoms,
or a cycloalkyl group of 3 or 4 carbon atoms which is optionally substituted by one or more R⁵ groups.

5. A compound according to Claim 4 in which R¹ is selected from a member of the group consisting of methyl, isopropyl, cyclopropyl and 1-methylcyclopropyl.

6. A compound according to any one of the preceding claims in which R² represents :-
a halogen atom, or
an alkyl group containing up to 6 carbons which is substituted by -OR⁵, or
a group selected from R⁵, nitro, -CO₂R³, -SOR⁵, -SR⁵, -SO₂R⁵, and -OR⁵.

7. A compound according to any one of the preceding claims in which R³ represents :-
a hydrogen atom, or
a straight or branched- chain alkyl group containing up to 6 carbon atoms.

8. A compound according to any one of the preceding claims in which R⁴ represents a hydrogen atom.

9. A compound according to any one of the preceding claims in which R⁵ represents a straight- or branched- chain alkyl group containing up to 6 carbon atoms which is optionally substituted by one or more halogen atoms.

10. A compound according to claim 1 wherein:
R represents a group -CO₂R³ or -COR⁵;
R¹ represents a straight- or branched- chain alkyl group containing up to 6 carbon atoms, or a cycloalkyl group containing 3 or 4 carbon atoms optionally substituted by one or more groups R⁵;
R² represents a halogen atom; or
an alkyl group containing up to 6 carbon atoms which is substituted by -OR⁵; or
a group R⁵, - NO₂, -CO₂R³, -SR⁵, -SOR⁵, - SO₂R⁵, -OR⁵, or
a group -O(CH₂)_{q}-OR⁵;
R³ represents a straight- or branched- chain alkyl group containing up to 6 carbon atoms;
R³¹ represents hydrogen;
R⁴ represents hydrogen;
R⁵ represents a straight- or branched- chain alkyl group containing up to 3 carbon atoms optionally substituted by one or more halogen atoms;
one of the groups R² is in the ortho position of the phenyl ring;
and n represents 2 or 3.

11. A compound according to claim 1 which is:
3-Methyl-4-(2-nitro-4-trifluoromethylbenzoyl)-isoxazole;
3-(1-Methylethyl)-4-(2-nitro-4-trifluoromethylbenzoyl)-isoxazole;
Ethyl 4-(2-nitro-4-trifluoromethylbenzoyl)-isoxazole-3-carboxylate;
3-Bromo-4-(2-nitro-4-trifluoromethylbenzoyl)-isoxazole;
4-(2-Nitro-4-trifluoromethylbenzoyl)-3-trifluoromethylisoxazole;
5-Cyclopropyl-3-methyl-4-(2-nitro-4-trifluoromethylbenzoyl)-isoxazole;
Ethyl 5-methyl-4-(2-nitro-4-trifluoromethylbenzoyl)-isoxazole-3-carboxylate;
5-Methyl-4-(2-nitro-4-trifluoromethylbenzoyl)-isoxazole-3-carboxamide;
Ethyl 5-cyclopropyl-4-(2-nitro-4-trifluoromethylbenzoyl)-isoxazole-3-carboxylate;
3-Cyano-5-methyl-4-(2-nitro-4-trifluoromethylbenzoyl)-isoxazole; or
Ethyl 4-(2-chloro-4-methylsulphonylbenzoyl)-5-cyclopropylisoxazole-3-carboxylate.

12. A compound according to claim 1 which is:
Ethyl 5-cyclopropyl-4-(4-methylsulphonyl-2-trifluoromethylbenzoyl) isoxazole-3-carboxylate;
Methyl 4-(2-chloro-4-methylsulphonylbenzoyl)-5-cyclopropylisoxazole-3-carboxylate.
4-(2-chloro-4-methylsulphonylbenzoyl)-5-cyclopropylisoxazole-3-carboxamide;
Ethyl 5-cyclopropyl-4-(2-methylsulphonylbenzoyl)-isoxazole-3-carboxylate;
Methyl 5-cyclopropyl-4-(4-methylsulphonyl-2-trifluoromethylbenzoyl)-isoxazole-3-carboxylate;
Ethyl 5-cyclopropyl-4-(2-nitro-4-methylsulphonylbenzoyl)-isoxazole-3-carboxylate;
Ethyl 5-cyclopropyl-4-(2,3-dichloro-4-methylsulphonylbenzoyl)-isoxazole-3-carboxylate;
Ethyl 4-(2-chloro-3-methoxy-4-methylsulphonylbenzoyl)-5-cyclopropylisoxazole-3-carboxylate;
4-(2-chloro-4-methylsulphonylbenzoyl)-3-cyano-5-cyclopropylisoxazole;
Ethyl 4-(4-chloro-2-methylsulphonylbenzoyl)-5-cyclopropylisoxazole-3-carboxylate;
Ethyl 4-(4-chloro-2-nitrobenzoyl)-5-cyclopropylisoxazole-3-carboxylate;
Ethyl 4-(4-chloro-2-trifluoromethylbenzoyl)-5-cyclopropylisoxazole-3-carboxylate;
Ethyl 5-cyclopropyl-4-[2-methylsulphenyl-4-trifluoromethylbenzoyl]-isoxazole-3-carboxylate;
Ethyl 5-cyclopropyl-4-[4-methysulphenyl-2-trifluoromethylbenzoyl]-isoxazole-3-carboxylate;
Ethyl 5-cyclopropyl-4-(2-methylsulphinyl-4-trifluoromethylbenzoyl)-isoxazole-3-carboxylate;
Ethyl 5-cyclopropyl-4-(4-methylsulphinyl-2-trifluoromethylbenzoyl)-isoxazole-3-carboxylate;
Ethyl 5-cyclopropyl-4-(2-methylsulphonyl-4-trifluoromethylbenzoyl)-isoxazole-3-carboxylate;
Ethyl 5-cyclopropyl-4-(2-fluoro-4-methylsulphonylbenzoyl)-isoxazole-3-carboxylate;
Ethyl 4-(2-chloro-4-ethylsulphonylbenzoyl)-5-cyclopropylisoxazole-3-carboxylate;
Ethyl 4-(4-bromo-2-methylsulphonylbenzoyl)-5-cyclopropylisoxazole-3-carboxylate;
Ethyl 4-(2-bromo-4-methylsulphonylbenzoyl)-5-cyclopropylisoxazole-3-carboxylate;
Ethyl 4-[2-chloro-4-methylsulphenyl-benzoyl]-5-cyclopropylisoxazole-3-carboxylate;
Ethyl 4-(2-chloro-4-methylsulphinylbenzoyl)-5-cyclopropylisoxazole-3-carboxylate;
3-Acetyl-4-(2-chloro-4-methylsulphonylbenzoyl)-5-cyclopropyl isoxazole;
Isopropyl 4-(2-chloro-4-methylsulphonylbenzoyl)-5-cyclopropylisoxazole-3-carboxylate;
Methyl 4-(4-chloro-2-methylsulphonylbenzoyl)-5-cyclopropylisoxazole-3-carboxylate;
Methyl 5-cyclopropyl-4-(2-methylsulphonyl-4-trifluoromethylbenzoyl)-isoxazole-3-carboxylate;
Methyl 4-[4-chloro-2-methylsulphenyl-benzoyl]-5-cyclopropylisoxazole-3-carboxylate; or
Methyl 4-(4-chloro-2-methylsulphinylbenzoyl)-5-cyclopropylisoxazole-3-carboxylate.

13. A compound according to claim 1 which is:
Ethyl 5-cyclopropyl-4-(2-methylsulphonyl-4-trifluoromethylbenzoyl)-isoxazole-3-carboxylate.

14. A process for preparing a 4-benzoyl isoxazole derivative according to claim 1 which comprises:
a) the reaction of a compound of general formula II: 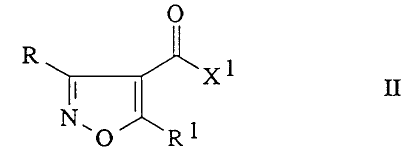 wherein R and R¹ are as defined in claim 1 and X¹ represents a halogen atom, with a compound of the general formula III: 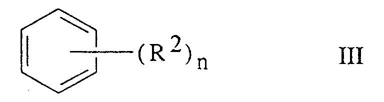 wherein R² and n are as defined in claim 1, in the presence of a Lewis acid catalyst;
b) the reaction of a compound of formula IV: 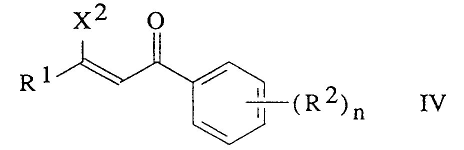 wherein R¹, R² and n are as defined in claim 1 and X² represents a group of the formula -N(R⁷)₂ or -SR⁷, wherein R⁷ represents a straight- or branched-chain alkyl group of up to 4 carbon atoms, with a compound of formula:
R-C(X¹)=N-OH
wherein R is as defined in claim 1 and X¹ represents a halogen atom, in the presence of an organic base in an inert solvent;
c) the reaction of a compound of formula VI: 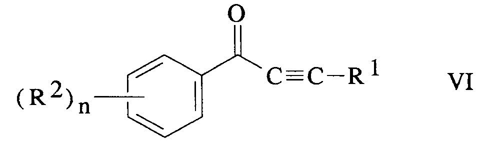 wherein R¹, R² and n are as defined in claim 1, with a compound of formula :
R-C(X¹)=N-OH
wherein R is as defined in claim 1 and X¹ represents a halogen atom, in the presence of an organic base or a catalyst in an inert solvent;
d) the oxidation of a compound of general formula VII: 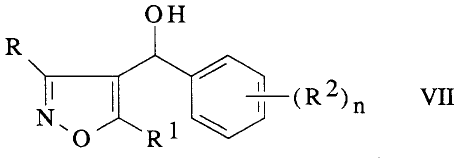 wherein R, R¹, R², and n are as defined in claim 1, to convert the hydroxy group to a ketone group;
e) the metallation of a compound of formula VIII: 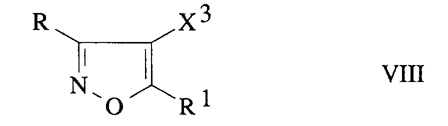 wherein R and R¹ are as defined in claim 1 and X³ represents bromine or iodine, followed by treatment with a benzoyl chloride of general formula IX: 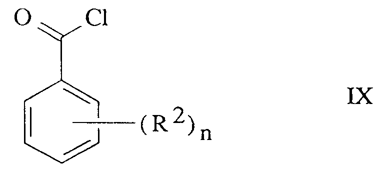 wherein R² and n are as hereinbefore defined;
f) the reaction of a salt of a compound of formula X: 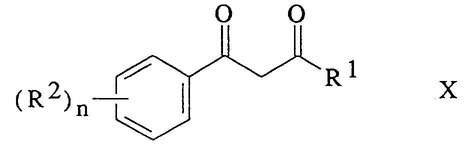 wherein R¹, R² and n are as defined in claim 1, with a compound of general formula:
R-C(X¹)=N-OH
wherein R is as defined in claim 1 and X¹ represents a halogen atom;
and optionally converting a 4-benzoyl isoxazole derivative of formula I thus obtained into an agriculturally acceptable salt thereof.

15. A herbicidal composition comprising as active ingredient a herbicidally effective amount of a 4-benzoyl isoxazole derivative of formula I as shown in claim 1 wherein:
R represents :-
a straight- or branched-chain alkyl, alkenyl or alkynyl group containing up to 6 carbon atoms which is optionally substituted by one or more halogen atoms; or
a cycloalkyl group containing from 3 to 6 carbon atoms optionally substituted by one or more substituents selected from groups R⁵, one or more halogen atoms and a group -CO₂R³; or
a group selected from -CO₂R³, -COR⁵, cyano, nitro, -CONR³¹R⁴,
or a halogen atom;
R¹ represents :-
a hydrogen atom, or
a straight- or branched-chain alkyl, alkenyl or alkynyl group containing up to 6 carbon atoms which is optionally substituted by one or more halogen atoms; or
a cycloalkyl group containing from 3 to 6 carbon atoms optionally substituted by one or more substituents selected from groups R⁵ and one or more halogen atoms;
R² represents :-
a halogen atom, or
a group selected from R⁵, -SR⁵, -SOR⁵, -SO₂R⁵, -SO₂NR³¹R⁴, -CO₂R³, -COR⁵, -CONR³¹R⁴, -CSNR³¹R⁴, -OR⁵, a nitro group, a cyano group, a group -O(CH₂)_{q}-OR⁵, or a straight- or branched- chain alkyl group containing up to 6 carbons and which is substituted by OR⁵;
R³, R³¹ and R⁴, which may be the same or different, each represents :-
a hydrogen atom, or
a straight- or branched-chain alkyl group containing up to 6 carbon atoms which is optionally substituted by one or more halogen atoms;
R⁵ represents :-
a straight- or branched-chain alkyl group containing up to 6 carbon atoms which is optionally substituted by one or more halogen atoms;
n represents an integer from 1 to 5;
q represents an integer from 1 to 3;
or an agriculturally acceptable salt thereof; in association with a herbicidally acceptable diluent or carrier and/or herbicidally acceptable surface- active agent.

16. A herbicidal composition comprising as active ingredient a herbicidally effective amount of a compound according to any one of claims 1 to 13 in association with a herbicidally acceptable diluent or carrier and/or herbicidally acceptable surface- active agent.

17. A herbicidal composition according to claim 15 or 16 which comprises 0.05 to 90% by weight of active ingredient.

18. A herbicidal compositions according to any one of claims 15 to 17 which is in liquid form and contains 0.05 to 25% by weight of surface-active agent.

19. A herbicidal composition according to any one of claims 15 to 17 in the form of an aqueous suspension concentrate or of a wettable powder or of a water soluble or water dispersible powder or of a liquid water soluble or water dispersible concentrate or of a liquid emulsifiable suspension or of a granule.

20. A herbicidal composition according to claim 19 wherein the composition is in the form of an aqueous suspension containing 10 to 70% of active ingredient or of a wettable powder containing 10 to 90% of active ingredient or of a water dispersible or water soluble powder containing 10 to 90% of active ingredient or of a liquid water soluble concentrate containing 5 to 50% of active ingredient or of a liquid emulsifiable suspension concentrate containing 10 to 70% of active ingredient or of granules containing 1 to 90% of active ingredient, the percentages being by weight.

21. A herbicidal composition according to claim 19 or 20 wherein the composition is in the form of an aqueous suspension containing 2 to 10% of surface-active agent or of a wettable powder containing 2 to 10% of surface-active agent or of a liquid emulsifiable suspension concentrate containing 5 to 15% of surface-active agent or of a liquid water soluble concentrate containing 5 to 25% of surface-active agent or of granules containing 0.5 to 7% of surface-active agent, the percentages being by weight.

22. A method of controlling the growth of weeds at a locus which comprises applying thereto a herbicidally effective amount of a 4-benzoyl isoxazole derivative according to any one of claims 1 to 13 or 15, or an agriculturally acceptable salt thereof.

23. A method according to claim 22 wherein the locus is an area used, or to be used, for growing crops, using an application rate of between 0.01 kg and 4.0 kg of active material per hectare.

24. A method according to claim 22 wherein the locus is not a crop-growing area, using an application rate of between 1.0 kg and 20.0 kg of active material per hectare.

## Claims (Claims for the following Contracting State(s): ES)

1. A process for the preparation of a 4-benzoyl isoxazole derivative of formula I: 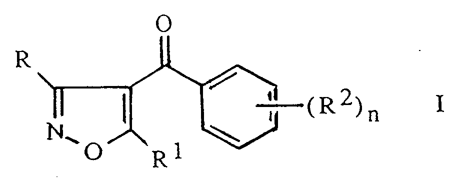 wherein:
R represents:-
a straight- or branched-chain alkyl, alkenyl or alkynyl group containing up to 6 carbon atoms which is optionally substituted by one or more halogen atoms; or
a cycloalkyl group containing from 3 to 6 carbon atoms optionally substituted by one or more substituents selected from groups R⁵, one or more halogen atoms and a group -CO₂R³; or
a group selected from -CO₂R³, -COR⁵, cyano, nitro, -CONR³¹R⁴,
or a halogen atom;
R¹ represents:-
a hydrogen atom, or
a straight- or branched-chain alkyl, alkenyl or alkynyl group containing up to 6 carbon atoms which is optionally substituted by one or more halogen atoms; or
a cycloalkyl group containing from 3 to 6 carbon atoms optionally substituted by one or more substituents selected from groups R⁵ and one or more halogen atoms;
R² represents:-
a halogen atom, or
a group selected from R⁵, -SR⁵, -SOR⁵, -SO₂R⁵, -SO₂NR³¹R⁴, -CO₂R³, -COR⁵, -CONR³¹R⁴, -CSNR³¹R⁴, -OR⁵, a nitro group, a cyano group, a group -O(CH₂)_{q}-OR⁵, or a straight-or branched-chain alkyl group containing up to 6 carbons and which is substituted by OR⁵;
R³, R³¹ and R⁴, which may be the same or different, each represents:-
a hydrogen atom, or
a straight- or branched-chain alkyl group containing up to 6 carbon atoms which is optionally substituted by one or more halogen atoms;
R⁵ represents:-
a straight- or branched-chain alkyl group containing up to 6 carbon atoms which is optionally substituted by one or more halogen atoms;
n represents an integer from 1 to 5;
q represents an integer from 1 to 3;
provided that R and R¹ do not simultaneously represent a methyl group; or an agriculturally acceptable salt thereof,
which process comprises:
a) the reaction of a compound of general formula II: 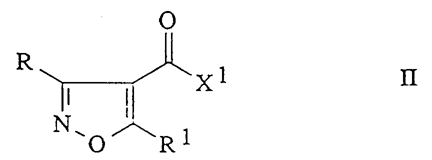 wherein R and R¹ are as hereinbefore defined and X¹ represents a halogen atom, with a compound of the general formula III: 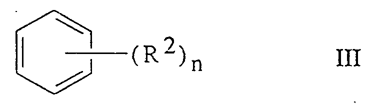 wherein R² and n are as hereinbefore defined, in the presence of a Lewis acid catalyst;
b) the reaction of a compound of formula IV: 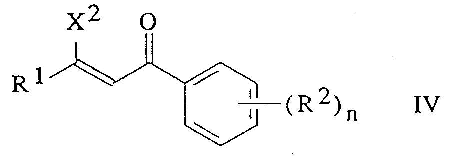 wherein R¹, R² and n are as hereinbefore defined and X² represents a group of the formula -N(R⁷)₂ or -SR⁷, wherein R⁷ represents a straight- or branched-chain alkyl group of up to 4 carbon atoms, with a compound of formula:
R-C(X¹)=N-OH
wherein R is as hereinbefore defined and X¹ represents a halogen atom, in the presence of an organic base in an inert solvent;
c) the reaction of a compound of formula VI: 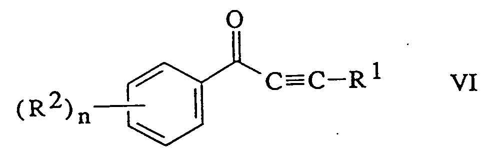 wherein R¹, R² and n are as hereinbefore defined, with a compound of formula:
R-C(X¹)=N-OH
wherein R is as hereinbefore defined and X¹ represents a halogen atom, in the presence of an organic base or a catalyst in an inert solvent;
d) the oxidation of a compound of general formula VII: 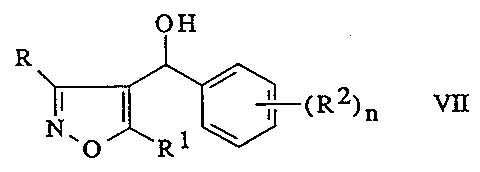 wherein R, R¹, R², and n are as hereinbefore defined, to convert the hydroxy group to a ketone group;
e) the metallation of a compound of formula VIII: 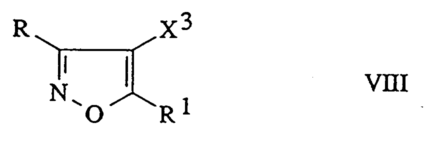 wherein R and R¹ are as hereinbefore defined and X³ represents bromine or iodine, followed by treatment with a benzoyl chloride of general formula IX: 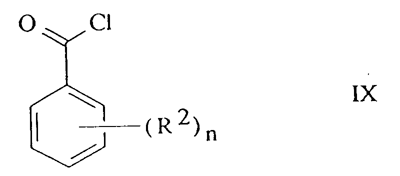 wherein R² and n are as hereinbefore defined;
f) the reaction of a salt of a compound of formula X: 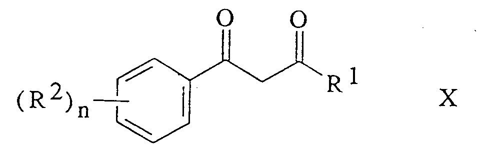 wherein R¹, R² and n are as hereinbefore defined, with a compound of general formula:
R-C(X¹)=N-OH
wherein R is as hereinbefore defined and X¹ represents a halogen atom;
and optionally converting a 4-benzoyl isoxazole derivative of formula I thus obtained into an agriculturally acceptable salt thereof.

2. A process according to claim 1 for the preparation of a compound in which at least one R² occupies an ortho position adjacent to the carbonyl group linking the phenyl and isoxazolyl rings.

3. A process according to claim 1 or 2 in which R represents:-
a straight- or branched- chain alkyl group containing up to 6 carbon atoms which is optionally substituted by one or more halogen atoms, or
a cycloalkyl group containing from 3 to 6 carbon atoms, or
a group selected from -COR⁵, -CO₂R³, or
a halogen atom.

4. A process according to claim 1, 2 or 3 in which R¹ represents:-
a straight- or branched- chain alkyl group containing up to 6 carbon atoms,
or a cycloalkyl group of 3 or 4 carbon atoms which is optionally substituted by one or more R⁵ groups.

5. A process according to Claim 4 in which R¹ is selected from a member of the group consisting of methyl, isopropyl, cyclopropyl and 1-methylcyclopropyl.

6. A process according to any one of the preceding claims in which R² represents:-
a halogen atom, or
an alkyl group containing up to 6 carbons which is substituted by -OR⁵, or
a group selected from R⁵, nitro, -CO₂R³, -SOR⁵, -SR⁵, SO₂R⁵, and -OR⁵.

7. A process according to any one of the preceding claims in which
R³ represents:-
a hydrogen atom, or
a straight or branched- chain alkyl group containing up to 6 carbon atoms.

8. A process according to any one of the preceding claims in which R⁴ represents a hydrogen atom.

9. A process according to any one of the preceding claims in which R⁵ represents a straight- or branched- chain alkyl group containing up to 6 carbon atoms which is optionally substituted by one or more halogen atoms.

10. A process according to claim 1 wherein:
R represents a group -CO₂R³ or COR⁵;
R¹ represents a straight- or branched- chain alkyl group containing up to 6 carbon atoms, or a cycloalkyl group containing 3 or 4 carbon atoms optionally substituted by one or more groups R⁵;
R² represents a halogen atom; or
an alkyl group containing up to 6 carbon atoms which is substituted by -OR⁵; or
a group R⁵, -NO₂, -CO₂R³, -SR⁵, -SOR⁵, SO₂R⁵, -OR⁵, or a group -O(CH₂)_{q}-OR⁵;
R³ represents a straight- or branched- chain alkyl group containing up to 6 carbon atoms;
R³¹ represents hydrogen;
R⁴ represents hydrogen;
R⁵ represents a straight- or branched- chain alkyl group containing up to 3 carbon atoms optionally substituted by one or more halogen atoms;
one of the groups R² is in the ortho position of the phenyl ring;
and n represents 2 or 3.

11. A process according to claim 1 for the preparation of:
3-Methyl-4-(2-nitro-4-trifluoromethylbenzoyl)-isoxazole;
3-(1-Methylethyl)-4-(2-nitro-4-trifluoromethylbenzoyl)-isoxazole;
Ethyl 4-(2-nitro-4-trifluoromethylbenzoyl)-isoxazole-3-carboxylate;
3-Bromo-4-(2-nitro-4-trifluoromethylbenzoyl)-isoxazole;
4-(2-Nitro-4-trifluoromethylbenzoyl)-3-trifluoromethylisoxazole;
5-Cyclopropyl-3-methyl-4-(2-nitro-4-trifluoromethylbenzoyl)-isoxazole;
Ethyl 5-methyl-4-(2-nitro-4-trifluoromethylbenzoyl)-isoxazole-3-carboxylate;
5-Methyl-4-(2-nitro-4-trifluoromethylbenzoyl)-isoxazole-3-carboxamide;
Ethyl 5-cyclopropyl-4-(2-nitro-4-trifluoromethylbenzoyl)-isoxazole-3-carboxylate,
3-Cyano-5-methyl-4-(2-nitro-4-trifluoromethylbenzoyl)-isoxazole; or
Ethyl 4-(2-chloro-4-methylsulphonylbenzoyl)-5-cyclopropylisoxazole-3-carboxylate.

12. A process according to claim 1 for the preparation of:
Ethyl 5-cyclopropyl-4-(4-methylsulphonyl-2-trifluoromethylbenzoyl) isoxazole-3-carboxylate;
Methyl 4-(2-chloro-4-methylsulphonylbenzoyl)-5-cyclopropylisoxazole-3-carboxylate.
4-(2-chloro-4-methylsulphonylbenzoyl)-5-cyclopropylisoxazole-3-carboxamide;
Ethyl 5-cyclopropyl-4-(2-methylsulphonylbenzoyl)-isoxazole-3-carboxylate;
Methyl 5-cyclopropyl-4-(4-methylsulphonyl-2-trifluoromethylbenzoyl)-isoxazole-3-carboxylate;
Ethyl 5-cyclopropyl-4-(2-nitro-4-methylsulphonylbenzoyl)-isoxazole-3-carboxylate;
Ethyl 5-cyclopropyl-4-(2,3-dichloro-4-methylsulphonylbenzoyl)-isoxazole-3-carboxylate;
Ethyl 4-(2-chloro-3-methoxy-4-methylsulphonylbenzoyl)-5-cyclopropylisoxazole-3-carboxylate;
4-(2-chloro-4-methylsulphonylbenzoyl)-3-cyano-5-cyclopropylisoxazole;
Ethyl 4-(4-chloro-2-methylsulphonylbenzoyl)-5-cyclopropylisoxazole-3-carboxylate;
Ethyl 4-(4-chloro-2-nitrobenzoyl)-5-cyclopropylisoxazole-3-carboxylate;
Ethyl 4-(4-chloro-2-trifluoromethylbenzoyl)-5-cyclopropylisoxazole-3-carboxylate;
Ethyl 5-cyclopropyl-4-[2-methylsulphenyl-4-trifluoromethylbenzoyl]-isoxazole-3-carboxylate;
Ethyl 5-cyclopropyl-4-[4-methysulphenyl-2-trifluoromethylbenzoyl]-isoxazole-3-carboxylate;
Ethyl 5-cyclopropyl-4-(2-methylsulphinyl-4-trifluoromethylbenzoyl)-isoxazole-3-carboxylate;
Ethyl 5-cyclopropyl-4-(4-methylsulphinyl-2-trifluoromethylbenzoyl)-isoxazole-3-carboxylate;
Ethyl 5-cyclopropyl-4-(2-methylsulphonyl-4-trifluoromethylbenzoyl)-isoxazole-3-carboxylate;
Ethyl 5-cyclopropyl-4-(2-fluoro-4-methylsulphonylbenzoyl)-isoxazole-3-carboxylate;
Ethyl 4-(2-chloro-4-ethylsulphonylbenzoyl)-5-cyclopropylisoxazole-3-carboxylate;
Ethyl 4-(4-bromo-2-methylsulphonylbenzoyl)-5-cyclopropylisoxazole-3-carboxylate;
Ethyl 4-(2-bromo-4-methylsulphonylbenzoyl)-5-cyclopropylisoxazole-3-carboxylate;
Ethyl 4-[2-chloro-4-methylsulphenyl-benzoyl]-5-cyclopropylisoxazole-3-carboxylate;
Ethyl 4-(2-chloro-4-methylsulphinylbenzoyl)-5-cyclopropylisoxazole-3-carboxylate;
3-Acetyl-4-(2-chloro-4-methylsulphonylbenzoyl)-5-cyclopropyl isoxazole;
Isopropyl 4-(2-chloro-4-methylsulphonylbenzoyl)-5-cyclopropylisoxazole-3-carboxylate;
Methyl 4-(4-chloro-2-methylsulphonylbenzoyl)-5-cyclopropylisoxazole-3-carboxylate;
Methyl 5-cyclopropyl-4-(2-methylsulphonyl-4-trifluoromethylbenzoyl)-isoxazole-3-carboxylate;
Methyl 4-[4-chloro-2-methylsulphenyl-benzoyl]-5-cyclopropylisoxazole-3-carboxylate; or
Methyl 4-(4-chloro-2-methylsulphinylbenzoyl)-5-cyclopropylisoxazole-3-carboxylate.

13. A process according to claim 1 for the preparation of:
Ethyl 5-cyclopropyl-4-(2-methylsulphonyl-4-trifluoromethylbenzoyl)-isoxazole-3-carboxylate.

14. A process for the preparation of a herbicidal composition which comprises formulating as active ingredient a herbicidally effective amount of a 4-benzoyl isoxazole derivative of formula I as shown in claim 1 wherein:
R represents :-
a straight- or branched-chain alkyl, alkenyl or alkynyl group containing up to 6 carbon atoms which is optionally substituted by one or more halogen atoms; or
a cycloalkyl group containing from 3 to 6 carbon atoms optionally substituted by one or more substituents selected from groups R⁵, one or more halogen atoms and a group -CO₂R³; or
a group selected from -CO₂R³, -COR⁵, cyano, nitro, -CONR³¹R⁴,
or a halogen atom;
R¹ represents :-
a hydrogen atom, or
a straight- or branched-chain alkyl, alkenyl or alkynyl group containing up to 6 carbon atoms which is optionally substituted by one or more halogen atoms; or
a cycloalkyl group containing from 3 to 6 carbon atoms optionally substituted by one or more substituents selected from groups R⁵ and one or more halogen atoms;
R² represents :-
a halogen atom, or
a group selected from R⁵, -SR⁵, -SOR⁵, -SO₂R⁵, -SO₂NR³¹R⁴, -CO₂R³, -COR⁵, -CONR³¹R⁴, -CSNR³¹R⁴, -OR⁵, a nitro group, a cyano group, a group -O(CH₂)_{q}-OR⁵, or a straight- or branched- chain alkyl group containing up to 6 carbons and which is substituted by OR⁵;
R³, R³¹ and R⁴, which may be the same or different, each represents :-
a hydrogen atom, or
a straight- or branched-chain alkyl group containing up to 6 carbon atoms which is optionally substituted by one or more halogen atoms;
R⁵ represents :-
a straight- or branched-chain alkyl group containing up to 6 carbon atoms which is optionally substituted by one or more halogen atoms;
n represents an integer from 1 to 5;
q represents an integer from 1 to 3;
or an agriculturally acceptable salt thereof; in association with a herbicidally acceptable diluent or carrier and/or herbicidally acceptable surface-active agent.

15. A process for the preparation of a herbicidal composition which comprises formulating as active ingredient a herbicidally effective amount of a compound according to any one of claims 1 to 13 in association with a herbicidally acceptable diluent or carrier and/or herbicidally acceptable surface- active agent.

16. A process according to claim 14 or 15 wherein the herbicidal composition comprises 0.05 to 90% by weight of active ingredient.

17. A process according to any one of claims 14 to 16 wherein the herbicidal composition is in liquid form and contains 0.05 to 25% by weight of surface-active agent.

18. A process according to any one of claims 14 to 16 wherein the herbicidal composition is in the form of an aqueous suspension concentrate or of a wettable powder or of a water soluble or water dispersible powder or of a liquid water soluble or water dispersible concentrate or of a liquid emulsifiable suspension or of a granule.

19. A process according to claim 18 wherein the herbicidal composition is in the form of an aqueous suspension containing 10 of 70% of active ingredient or of a wettable powder containing 10 to 90% of active ingredient or of a water dispersible or water soluble powder containing 10 to 90% of active ingredient or of a liquid water soluble concentrate containing 5 to 50% of active ingredient or of a liquid emulsifiable suspension concentrate containing 10 to 70% of active ingredient or of granules containing 1 to 90% of active ingredient, the percentages being by weight.

20. A process according to claim 18 or 19 wherein the herbicidal composition is in the form of an aqueous suspension containing 2 to 10% of surface-active agent or of a wettable powder containing 2 to 10% of surface-active agent or of a liquid emulsifiable suspension concentrate containing 5 to 15% of surface-active agent or of a liquid water soluble concentrate containing 5 to 25% of surface-active agent or of granules containing 0.5 to 7% of surface-active agent, the percentages being by weight.

21. A method of controlling the growth of weeds at a locus which comprises applying thereto a herbicidally effective amount of a 4-benzoyl isoxazole derivative as defined in any one of claims 1 to 14, or an agriculturally acceptable salt thereof.

22. A method according to claim 21 wherein the locus is an area used, or to be used, for growing crops, using an application rate of between 0.01 kg and 4.0 kg of active material per hectare.

23. A method according to claim 21 wherein the locus is not a crop-growing area, using an application rate of between 1.0 kg and 20.0 kg of active material per hectare.

24. A 4-benzoyl isoxazole derivative as defined in any one of claims 1 to 14.

25. A herbicidal composition as defined in any one of claims 14 to 20.

## Patentansprüche (Patentansprüche für folgende(n) Vertragsstaat(en): AT, BE, CH, LI, DE, DK, FR, GB, GR, IT, LU, NL, SE)

1. 4-Benzoylisoxazolderivate der Formel 1: 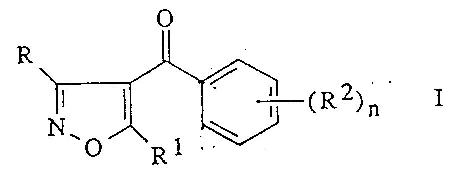 worin bedeuten:
R eine geradkettige oder verzweigte Alkyl-, Alkenyl- oder Alkinylgruppe, die bis zu 6 Kohlenstoffatome enthält und die ggfs. mit einem oder mehreren Halogenatomen substituiert ist, oder
eine Cycloalkylgruppe, die 3 bis 6 Kohlenstoffatome enthält, die ggfs. mit einem oder mehreren Substitutenten substituiert ist, die unter den Gruppen R⁵, einem oder mehreren Halogenatomen und unter einer Gruppe -CO₂R₃ ausgewählt sind, oder
eine Gruppe, die unter -CO₂R³ , -COR⁵, Cyano, Nitro, und -CONR³¹R⁴ ausgewählt ist, oder
ein Halogenatom;
R¹ ein Wasserstoffatom oder eine geradkettige oder verzweigte Alkyl-, Alkenyl- oder Aklkynylgruppe, die bis zu 6 Kohlenstoffatome enthält, die ggfs. mit einem oder mehreren Halogenatomen substituiert ist, oder
eine Cycloalkylgruppe, die 3 bis 6 Kohlenstoffatome enthält und die ggfs. mit einem oder mehreren Substitutenten substituiert ist, die unter den Gruppen R⁵, einem oder mehreren Halogenatomen und unter einer Gruppe -CO₂R₃ ausgewählt sind;
R² ein Halogenatom oder
eine Gruppe, die unter R⁵, -SR⁵, -SOR⁵, SO₂R⁵, -SO₂NR³¹R⁴, -CO₂R³, -COR⁵, -CONR³¹R⁴, -CSNR³¹R⁴, -OR⁵, einer Nitrogruppe, einer Cyanogruppe, einer Gruppe -O(CH₂)q-OR⁵ oder einer geradkettigen oder verzweigten Alkylgruppe ausgewählt ist, die bis zu 6 Kohlenstoffatome enthält und mit OR⁵ substituiert ist,
worin R³, R³¹ und R⁴ unabhängig gleich oder voneinander verschieden sind und bedeuten:
ein Wasserstoffatom oder
eine geradkettige oder verzweigte Alkylgruppe, die bis zu 6 Kohlenstoffatome enthält und die ggfs. mit einem oder mehreren Halogenatomen substituiert ist;
R⁵ eine geradkettige oder verzweigte Alkylgruppe, die bis zu 6 Kohlenstoffatome enthält und die ggfs. mit einem oder mehreren Halogenatomen substituiert ist;
n eine ganze Zahl von 1 bis 5;
q eine ganze Zahl von 1 bis 3
mit der Maßgabe, daß R und R¹ nicht gleichzeitig eine Methylgruppe bedeuten,
sowie für die Landwirtschaft akzeptable Salze davon.

2. Verbindungen nach Anspruch 1, bei denen mindestens ein Substituent R² eine ortho-Stellung einnimmt, die der Carbonylgruppe benachbart ist, welche den Phenylring mit dem Isoxazolylring verbindet.

3. Verbindungen nach Anspruch 1 oder 2, worin R bedeutet: eine geradkettige oder verzweigte Alkylgruppe, die bis zu 6 Kohlenstoffatome enthält und die ggfs. mit einem oder mehreren Halogenatomen substituiert ist, oder
eine Cycloalkylgruppe, die 3 bis 6 Kohlenstoffatome enthält, oder
eine Gruppe, die unter -COR⁵, -CO₂R ausgewählt ist, oder ein Halogenatom.

4. Verbindungen nach Anspruch 1 oder 2, worin R¹ bedeutet:
eine geradkettige oder verzweigte Alkylgruppe, die bis zu 6 Kohlenstoffatome enthält, oder
eine Cycloalkylgruppe mit 3 oder 4 Kohlenstoffatomen, die ggfs. mit einer oder mehreren Gruppen R⁵ substituiert ist.

5. Verbindungen nach Anspruch 4, worin R¹ unter Methyl, Isopropyl, Cyclopropyl und 1-Methylcyclopropyl ausgewählt ist.

6. Verbindungen nach einem der vorhergehenden Ansprüche, worin R² bedeutet:
ein Halogenatom oder
eine Alkylgruppe, die bis zu 6 Kohlenstoffatome enthält und mit -OR⁵ substituiert ist, oder
eine Gruppe, die unter R5, Nitro, -CO₂R³, -SOR⁵, SR⁵, -SO₂R⁵ und -OR⁵ ausgewählt ist.

7. Verbindungen nach einem der vorhergehenden Ansprüche, worin R³ bedeutet:
ein Wasserstoffatom oder
eine geradkettige oder verzweigte Alkylgruppe, die bis zu 6 Kohlenstoffatome enthält.

8. Verbindungen nach einem der vorhergehenden Ansprüche, worin R⁴ ein Wasserstoffatom bedeutet.

9. Verbindungen nach einem der vorhergehenden Ansprüche, worin R⁵ eine geradkettige oder verzweigte Alkylgruppe bedeutet, die bis zu 6 Kohlenstoffatomen enthält, die ggfs. mit einem oder mehreren Halogenatomen substituiert ist.

10. Verbindungen nach Anspruch 1, worin bedeuten:
R eine Gruppe -CO₂R³ oder -COR⁵;
R¹ eine geradkettige oder verzweigte Alkylgruppe, die bis zu 6 Kohlenstoffatome enthält, oder eine Cycloalkylgruppe, die 3 oder 4 Kohlenstoffatome enthält und die ggfs. mit einer oder mehrerer Gruppen R⁵ stubstituiert ist;
R² ein Halogenatom oder
eine Alkylgruppe, die bis zu 6 Kohlenstoffatome enthält, die mit -OR⁵ substituiert ist, oder
eine Gruppe R⁵, -NO², -CO₂R³, -SR⁵, -SOR⁵, -SO₂R⁵, -OR⁵ oder eine Gruppe -O(CH₂)q-OR⁵;
R³ eine geradkettige oder verzweigte Alkylgruppe, die bis zu 6 Kohlenstoffatome enthält;
R³¹ Wasserstoff;
R⁴ Wasserstoff;
R⁵ eine geradkettige oder verzweigte Alkylgruppe, die bis zu 5 Kohlenstoffatome enthält, die ggfs. mit einem oder mehreren Halogenatomen substitutiert ist;
eine der Gruppen R² am Phenylring in ortho-Stellung vorliegt, und
n 2 oder 3.

11. Verbindungen nach Anspruch 1:
3-Methyl-4-(2-nitro-4-trifluoromethylbenzoyl)-isoxazol;
3-(1-Methylethyl)-4-(2-nitro-4-trifluomethylbenzoyl)-isoxazol;
Ethyl-4-(2-nitro-4-trifluormethylbenzoyl)-isoxazol-3-carboxylat;
3-Brom-4-(2-nitro-4-trifluormethylbenzoyl)-isoxazol;
4-(2-Nitro-4-trifluormethylbenzoyl)-3-trifluormethylisoxazol;
5-Cyclopropyl-3-methyl-4-(2-nitro-4-trifluormethylbenzoyl)-isoxazol;
Ethyl-5-methyl-4-(2-nitro-4-trifluormethylbenzoyl)-isoxazol-3-carboxylat;
5-Methyl-4-(2-nitro-4-trifluormethylbenzoyl)-isoxazol-3-carboxamid;
Ethyl-5-cyclopropyl-4-(2-nitro-4-trifluormethylbenzoyl)ixoxazol-3-carboxylat;
3-Cyano-5-methyl-4-(2-nitro-4-trifluormethylbenzoyl)-isoxazol oder
Ethyl-4-(2-chlor-4-methylsulfonylbenzoyl)-5-cyclopropylisoxazol-3-carboxylat.

12. Verbindungen nach Anspruch 1:
Ethyl-5-cyclopropyl-4-(4-methylsulfonyl-2-trifluormethylbenzoyl)-isoxazol-3-carboxylat;
Methyl-4-(2-chlor-4-methylsulfonylbenzoyl)-5-cyclopropylisoxazol-3-carboxylat;
4-(2-chlor-4-methylsuphonylbenzoyl)-5-cyclopropylisoxazol-3-carboxamid;
Ethyl-5-cyclopropyl-4-(2-methylsulfonylbenzoyl)ixoxazol-3-carboxylat;
Methyl-5-cyclopropyl-4-methylsulfonyl-2-trifluormethyl-benzoyl)-isoxazol-3-carboxylat;
Ethyl-5-cyclopropyl-4-(2-nitro-4-methylsulfonylbenzoyl)-isoxazol-3-carboxylat;
Ethyl-5-cyclopropyl-4-(2,3-dichlor-4-methylsulfonylbenzoyl)-isocazol-3-carboxylat;
Ethyl-4-(2-chlor-3-methoxy-4-methylsulfonylbenzoyl)-5-cyclopropylisoxazol-3-carboxylat;
4-(2-chlor-4-methylsulfonylbenzoyl)-3-cyano-5-cyclopropylisoxazol;
Ethyl-4-(4-chlor-2-methylsulfonylbenzoyl)-5-cyclopropylisoxazol-3-carboxylat;
Ethyl-4-(4-chlor-2-nitrobenzoyl)-5-cyclopropylisoxazol-3-carboxylat;
Ethyl-4-(4-chlor-2-trifluormethylbenzoyl)-5-cyclopropylisoxazol-3-carboxylat;
Ethyl-5-cyclopropyl-4-(2-methylsulfenyl-4-trifluormethylbenzoyl)-isoxazol-3-carboxylat;
Ethyl-5-cyclopropyl-4-(4-methylsulfenyl-2-trifluormethylbenzoyl)-isoxazol-3-carboxylat;
Ethyl-5-cyclopropyl-4-(2-methylsulfinyl-4-trifluormethylbenzoyl)-isoxazol-3-carboxylat;
Ethyl-5-cyclopropyl-4-(4-methylsulfinyl-2-trifluormethylbenzoyl)-isoxazol-3-carboxylat;
Ethyl-5-cyclopropyl-4-(2-methylsulfonyl-4-trifluormethylbenzoyl)-isoxazol-3-carboxylat;
Ethyl-5-cyclopropyl-4-(2-fluor-4-methylsulfonylbenzoyl)isoxazol-3-carboxylat;
Ethyl-4-(2-chlor-4-ethylsulfonylbenzoyl)-5-cyclopropylisoxazol-3-carboxylat;
Ethyl-4-(4-brom-2-methylsulfonylbenzoyl)-5-cyclopropylisoxazol-3-carboxylat;
Ethyl-4-(2-brom-4-methylsulfonylbenzoyl)-5-cyclopropylisoxazol-3-carboxylat;
Ethyl-4-[2-chlor-4-methylsulfenylbenzoyl]-5-cyclopropylisoxazol-3-carboxylat;
Ethyl-4-(2-chlor-4-methylsulfinylbenzoyl)-5-cyclopropylisoxazol-3-carboxylat;
3-Acetyl-4-(2-chlor-4-methylsulfonylbenzoyl)-5-cyclopropylisoxazol;
Isopropyl-4-(2-chlor-4-methylsulfonylbenzoyl)-5-cyclopropylisoxazol-3-carboxylat;
Methyl-4-(4-chloro-2-methylsulfonylbenzoyl)-5-cyclopropylisoxazol-3-carboxylat;
Methyl-5-cyclopropyl-4-(2-methylsulfonyl-4-trifluormethylbenzoyl)-isoxazol-3-carboxylat;
Methyl-4-[4-chlor-2-methylsulfenylbenzoyl]-5-cyclopropylisoxazol-3-carboxylat oder
Methyl-4-(4-chlor-2-methylsulfinylbenzoyl)-5-cyclopropylixoxazol-3-carboxylat.

13. Verbindungen nach Anspruch 1:
Ethyl-5-cyclopropyl-4-(2-methylsulfonyl-4-trifluormethylbenzoyl)-isoxazol-3-carboxylat.

14. Verfahren zur Herstellung der 4-Benzoylisoxazolderivate nach Anspruch 1 mit den folgenden Schritten:
a) Umsetzung einer Verbindung der allgemeinen Formel II 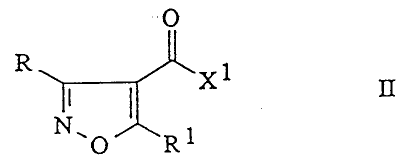 worin R und R¹ wie in Anspruch 1 definiert sind und X¹ ein Halogenatom bedeutet,
mit einer Verbindung der allgemeinen Formel III, 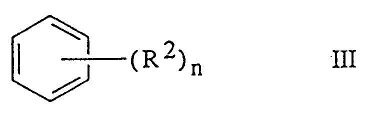 wobei R² und n wie in Anspruch 1 definiert sind, in Gegenwart einer Lewissäure als Katalysator;
b) Umsetzung einer Verbindung der Fomel IV 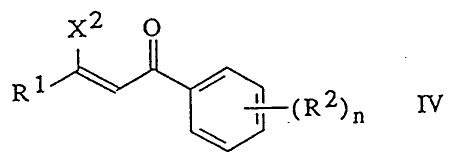 worin R¹, R² und n wie in Anspuch 1 definiert sind und X² eine Gruppe der Formel -N(R⁷)₂ oder -SR⁷ bedeuten, wobei R⁷ eine geradkettige oder verzweigte Alkylgruppe mit bis zu 4 Kohlenstoffatomen bedeutet,
mit einer Verbindung der Formel:
R-C(X¹) = N-OH ,
worin R wie in Anspruch 1 definiert ist und X¹ ein Halogenatom bedeutet,
in Gegenwart einer organischen Base in einem inerten Lösungsmittel;
c) Umsetzung einer Verbindung der Formel VI 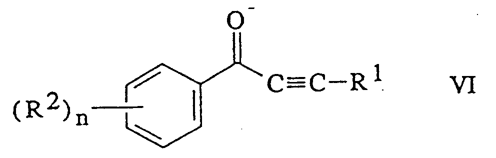 worin R¹, R² und n wie in Anspruch 1 definiert sind, mit einer Verbindung der Formel:
R-C(X¹) = N-OH ,
worin R wie in Anspruch 1 definiert ist und X¹ ein Halogenatom bedeutet,
in Gegenwart einer organischen Base oder eines Katalysators in einem inerten Lösungsmittel;
d) Oxidation einer Verbindung der allgemeinen Formel VII 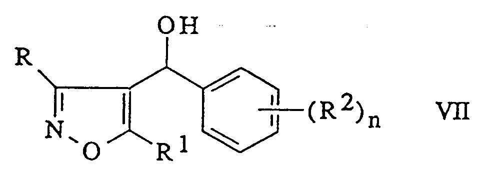 worin R, R¹, R² und n wie in Anspruch 1 definiert sind, um die Hydroxygruppe in eine Ketogruppe umzuwandeln;
e) Metallierung einer Verbindung der Formel VIII 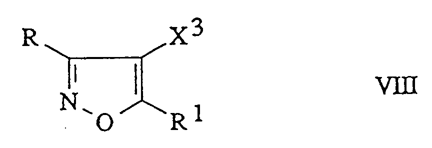 worin R und R¹ wie in Anspruch 1 definiert sind und X³ Brom oder Iod bedeutet,
worauf eine Behandlung mit einem Benzoylchlorid der allgemeinen Formen IX: 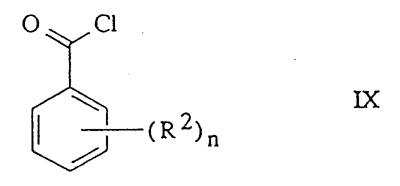 folgt, worin R² und n wie oben definiert sind;
f) Umsetzung eines Salzes einer Verbindung der Formel X 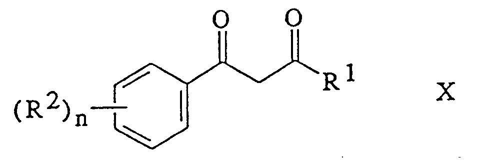 worin R, R¹ ,R² wie in Anspruch 1 definiert sind,
mit einer Verbindung der allgemeinen Formel
R-C(X¹) = N-OH,
worin R wie in Anspruch 1 definiert ist und X¹ ein Halogenatom bedeutet;
und wahlweise Umwandlung eines auf diese Weise erhaltenen 4-Benzoylisoxazolderivats der Formel I in ein für die Landwirtschaft akzeptables Salz davon.

15. Herbizide Zusammensetzung, die als Wirkstoff eine herbizid wirksame Menge eines 4-Benzoylisoxazolderivats der Formel I nach Anspruch 1 enthält,
worin bedeuten:
R eine geradkettige oder verzweigte Alkyl-, Alkenyl- oder Alkinylgruppe, die bis zu 6 Kohlenstoffatome enthält und die ggfs. mit einem oder mehreren Halogenatomen substituiert ist, oder
eine Cycloalkylgruppe, die 3 bis 6 Kohlenstoffatome enthält, die ggfs. mit einem oder mehreren Substitutenten substituiert ist, die unter den Gruppen R⁵, einem oder mehreren Halogenatomen und unter einer Gruppe -CO₂R₃ ausgewählt sind, oder
eine Gruppe, die unter -CO₂R³ , -COR⁵, Cyano, Nitro und -CONR³¹R⁴ ausgewählt ist, oder
ein Halogenatom;
R¹ ein Wasserstoffatom oder eine geradkettige oder verzweigte Alkyl-, Alkenyl- oder Alkinylgruppe, die bis zu 6 Kohlenstoffatome enthält, die ggfs. mit einem oder mehreren Halogenatomen substituiert ist, oder
eine Cycloalkylgruppe, die 3 bis 6 Kohlenstoffatome enthält und die ggfs. mit einem oder mehreren Substitutenten substituiert ist, die unter den Gruppen R⁵, einem oder mehreren Halogenatomen und unter einer Gruppe -CO₂R₃ ausgewählt sind;
R² ein Halogenatom oder
eine Gruppe, die unter R⁵, -SR⁵, -SOR⁵, SO₂R⁵, -SO₂NR³¹R⁴, - CO₂R³, -COR⁵, -CONR³¹R⁴, -CSNR³¹R⁴, -OR⁵, einer Nitrogruppe, einer Cyanogruppe, einer Gruppe -O(CH₂)q-OR⁵ oder einer geradkettigen oder verzweigten Alkylgruppe ausgewählt ist, die bis zu 6 Kohlenstoffatome enthält und mit OR⁵ substituiert ist;
worin R³, R³¹ und R⁴ gleich oder voneinander verschieden sind und bedeuten:
ein Wasserstoffatom oder
eine geradkettige oder verzweigte Alkylgruppe, die bis zu 6 Kohlenstoffatome enthält und die ggfs. mit einem oder mehreren Halogenatomen substituiert ist;
R⁵ eine geradkettige oder verzweigte Alkylgruppe, die bis zu 6 Kohlenstoffatome enthält und die ggfs. mit einem oder mehreren Halogenatomen substituiert ist;
n eine ganze Zahl von 1 bis 5;
q eine ganze Zahl von 1 bis 3
mit der Maßgabe, daß R und R¹ nicht gleichzeitig eine Methylgruppe bedeuten, oder
ein für die Landwirtschaft akzeptables Salz davon.

16. Herbizide Zusammensetzung, die als Wirkstoff eine herbizid wirksame Menge einer Verbindung nach einem der Ansprüche 1 bis 13 in Verbindung mit einem von der Herbizidwirkung akzeptablen Verdünnungsmittel oder einem Träger und/oder einem für Herbizide akzeptablen grenzflächenaktiven Mittel enthält.

17. Herbizide Zusammensetzung nach Anspruch 15 oder 16, die 0,05 bis 90 Gew.-% Wirkstoff enthält.

18. Herbizide Zusammensetzung nach einem der Ansprüche 15 bis 17, die in flüssiger Form vorliegt und 0,05 bis 25 Gew.-% grenzflächenaktives Mittel enthält.

19. Herbizide Zusammensetzung nach einem der Ansprüche 15 bis 17 in Form eines wäßrigen Suspensionskonzentrats oder eines Spritzpulvers oder eines wasserlöslichen oder in Wasser dispergierbaren Pulvers oder eines flüssigen wasserlöslichen oder in Wasser dispergierbaren Konzentrats oder einer flüssigen emulgierbaren Suspension oder eines Granulats.

20. Herbizide Zusammensetzung nach Anspruch 19, wobei die Zusammensetzung in Form
einer wäßrigen Suspension, die 10 bis 70 Gew.-% Wirkstoff enthält, oder
eines Spritzpulvers, das 10 bis 90 Gew.-% Wirkstoff enthält, oder
eines in Wasser dispergierbaren oder wasserlöslichen Pulvers, das 10 bis 90 Gew.-% Wirkstoff enthält, oder
eines flüssigen wasserlöslichen Konzentrats, das 5 bis 50 Gew.-% Wirkstoff enthält, oder
eines flüssigen emulgierbaren Suspensionskonzentrats, das 10 bis 70 Gew.-% Wirkstoff enthält, oder
eines Granulats vorliegt, das 1 bis 90 Gew.-% Wirkstoff enthält.

21. Herbizide Zusammensetzung nach Anspruch 19 oder 20, die Zusammensetzung in Form einer wäßrigen Suspension, die 2 bis 10 Gew.-% grenzflächenaktives Mittel enthält, oder eines Spritzpulvers, das 2 bis 10 Gew.-% grenzflächenaktives Mittel enthält, oder eines flüssigen emulgierbaren Suspensionskonzentrats, das 5 bis 15 Gew.-% grenzflächenaktives Mittel enthält, oder eines flüssigen wasserlöslichen Konzentrats, das 5 bis 25 Gew.-% grenzflächenaktives Mittel enthält, oder eines Granulats vorliegt, das 0,5 bis 7 Gew.-% grenzflächenaktives Mittel enthält.

22. Verfahren zur lokalen Kontrolle des Wachstums von Unkräutern, das das Ausbringen einer herbizid wirksamen Menge eines 4-Benzoylisoxazolderivats nach einem der Ansprüche 1 bis 13 oder 15 oder eines in der Landwirtschaft akzeptablen Salzes davon am betreffenden Ort umfaßt.

23. Verfahren nach Anspruch 22, wobei der Ort eine genutzte oder zu nutzende Anbaufläche ist und die Ausbringmenge 0,01 bis 4,0 kg Wirkstoff pro Hektar beträgt.

24. Verfahren nach Anspruch 22, wobei der Ort keine Anbaufläche ist und die Ausbringmenge 1,0 bis 20,0 kg Wirkstoff pro Hektar beträgt.

## Patentansprüche (Patentansprüche für folgende(n) Vertragsstaat(en): ES)

1. Verfahren zur Herstellung von 4-Benzoylisoxazolderivaten der Formel I: 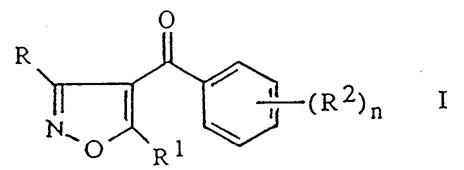 worin bedeuten:
R eine geradkettige oder verzweigte Alkyl-, Alkenyl- oder Alkinylgruppe, die bis zu 6 Kohlenstoffatome enthält und die ggfs. mit einem oder mehreren Halogenatomen substituiert ist, oder
eine Cycloalkylgruppe, die 3 bis 6 Kohlenstoffatome enthält, die ggfs. mit einem oder mehreren Substitutenten substituiert ist, die unter den Gruppen R⁵, einem oder mehreren Halogenatomen und unter einer Gruppe -CO₂R₃ ausgewählt sind, oder
eine Gruppe, die unter -CO₂R³ , -COR⁵, Cyano, Nitro, und -CONR³¹R⁴ ausgewählt ist, oder
ein Halogenatom;
R¹ ein Wasserstoffatom oder eine geradkettige oder verzweigte Alkyl-, Alkenyl- oder Aklkynylgruppe, die bis zu 6 Kohlenstoffatome enthält, die ggfs. mit einem oder mehreren Halogenatomen substituiert ist, oder
eine Cycloalkylgruppe, die 3 bis 6 Kohlenstoffatome enthält und die ggfs. mit einem oder mehreren Substitutenten substituiert ist, die unter den Gruppen R⁵, einem oder mehreren Halogenatomen und unter einer Gruppe -CO₂R₃ ausgewählt sind;
R² ein Halogenatom oder
eine Gruppe, die unter R⁵, -SR⁵, -SOR⁵, SO₂R⁵, -SO₂NR³¹R⁴, -CO₂R³, -COR⁵, -CONR³¹R⁴, -CSNR³¹R⁴, -OR⁵, einer Nitrogruppe, einer Cyanogruppe, einer Gruppe -O(CH₂)q-OR⁵ oder einer geradkettigen oder verzweigten Alkylgruppe ausgewählt ist, die bis zu 6 Kohlenstoffatome enthält und mit OR⁵ substituiert ist,
worin R³, R³¹ und R⁴ unabhängig gleich oder voneinander verschieden sind und worin bedeuten:
ein Wasserstoffatom oder
eine geradkettige oder verzweigte Alkylgruppe, die bis zu 6 Kohlenstoffatome enthält und die ggfs. mit einem oder mehreren Halogenatomen substituiert ist;
R⁵ eine geradkettige oder verzweigte Alkylgruppe, die bis zu 6 Kohlenstoffatome enthält und die ggfs. mit einem oder mehreren Halogenatomen substituiert ist;
n eine ganze Zahl von 1 bis 5;
q eine ganze Zahl von 1 bis 3
mit der Maßgabe, daß R und R¹ nicht gleichzeitig eine Methylgruppe bedeuten,
sowie für die Landwirtschaft akzeptable Salze davon, mit den folgenden Schritten:
a) Umsetzung einer Verbindung der allgemeinen Formel II 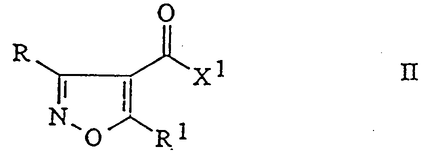 worin R und R¹ wie in Anspruch 1 definiert sind und X¹ ein Halogenatom bedeutet,
mit einer Verbindung der allgemeinen Formel III, 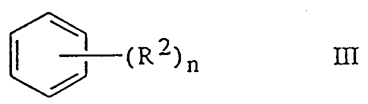 wobei R² und n wie in Anspruch 1 definiert sind,
in Gegenwart einer Lewissäure als Katalysator;
b) Umsetzung einer Verbindung der Fomel IV 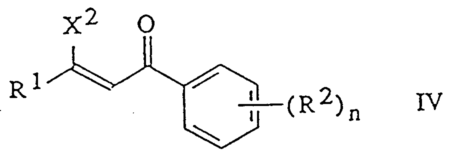 worin R¹, R² und n wie in Anspuch 1 definiert sind und X² eine Gruppe der Formel -N(R⁷)₂ oder -SR⁷ bedeuten, wobei R⁷ eine geradkettige oder verzweigte Alkylgruppe mit bis zu 4 Kohlenstoffatomen bedeutet,
mit einer Verbindung der Formel:
R-C(X¹) = N-OH ,
worin R wie in Anspruch 1 definiert ist und X¹ ein Halogenatom bedeutet,
in Gegenwart einer organischen Base in einem inerten Lösungsmittel;
c) Umsetzung einer Verbindung der Formel VI 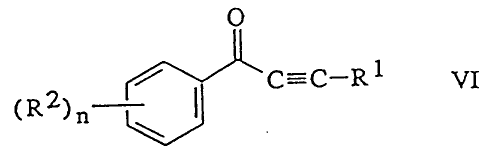 worin R¹, R² und n wie in Anspruch 1 definiert sind, mit einer Verbindung der Formel:
R-C(X¹) = N-OH ,
worin R wie in Anspruch 1 definiert ist und X¹ ein Halogenatom bedeutet,
in Gegenwart einer organischen Base oder eines Katalysators in einem inerten Lösungsmittel;
d) Oxidation einer Verbindung der allgemeinen Formel VII 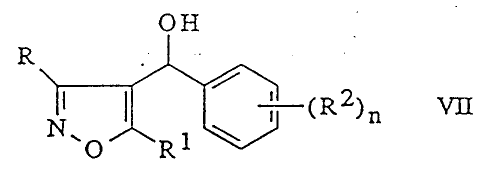 worin R, R¹, R² und n wie in Anspruch 1 definiert sind, um die Hydroxygruppe in eine Ketogruppe umzuwandeln;
e) Metallierung einer Verbindung der Formel VIII 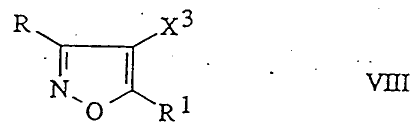 worin R und R¹ wie in Anspruch 1 definiert sind und X³ Brom oder Iod bedeutet,
worauf eine Behandlung mit einem Benzoylchlorid der allgemeinen Formen IX: 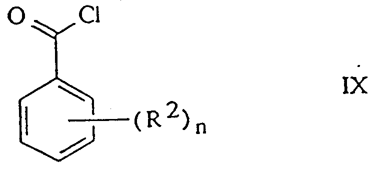 folgt, worin R² und n wie oben definiert sind;
f) Umsetzung eines Salzes einer Verbindung der Formel X 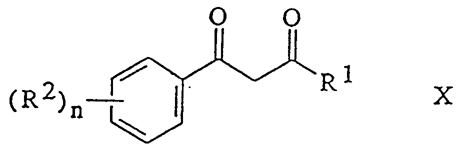 worin R, R¹ ,R² wie in Anspruch 1 definiert sind,
mit einer Verbindung der allgemeinen Formel
R-C(X¹) = N-OH,
worin R wie in Anspruch 1 definiert ist und X¹ ein Halogenatom bedeutet;
und wahlweise Umwandlung eines auf diese Weise erhaltenen 4-Benzoylisoxazolderivats der Formel I in ein für die Landwirtschaft akzeptables Salz davon.

2. Verfahren nach Anspruch 1 zur Herstellung von Verbindungen, bei denen mindestens ein Substituent R² eine ortho-Stellung einnimmt, die der Carbonylgruppe benachbart ist, welche den Phenylring mit dem Isoxazolylring verbindet.

3. Verfahren nach Anspruch 1 oder 2, worin R bedeutet:
eine geradkettige oder verzweigte Alkylgruppe, die bis zu 6 Kohlenstoffatome enthält und die ggfs. mit einem oder mehreren Halogenatomen substituiert ist, oder
eine Cycloalkylgruppe, die 3 bis 6 Kohlenstoffatome enthält, oder
eine Gruppe, die unter -COR⁵, -CO₂R³ ausgewählt ist, oder ein Halogenatom.

4. Verfahren nach Anspruch 1, 2 oder 3, worin R¹ bedeutet:
eine geradkettige oder verzweigte Alkylgruppe, die bis zu 6 Kohlenstoffatome enthält, oder
eine Cycloalkylgruppe mit 3 oder 4 Kohlenstoffatomen, die ggfs. mit einer oder mehreren Gruppen R⁵ substituiert ist.

5. Verfahren nach Anspruch 4, worin R¹ unter Methyl, Isopropyl, Cyclopropyl und 1-Methylcyclopropyl ausgewählt ist.

6. Verfahren nach einem der vorhergehenden Ansprüche, worin R² bedeutet:
ein Halogenatom oder
eine Alkylgruppe, die bis zu 6 Kohlenstoffatome enthält und mit -OR⁵ substituiert ist, oder
eine Gruppe, die unter R5, Nitro, -CO₂R³, -SOR⁵, SR⁵, -SO₂R⁵ und -OR⁵ ausgewählt ist.

7. Verfahren nach einem der vorhergehenden Ansprüche, worin R³ bedeutet:
ein Wasserstoffatom oder
eine geradkettige oder verzweigte Alkylgruppe, die bis zu 6 Kohlenstoffatome enthält.

8. Verfahren nach einem der vorhergehenden Ansprüche, worin R⁴ ein Wasserstoffatom bedeutet.

9. Verfahren nach einem der vorhergehenden Ansprüche, worin R⁵ eine geradkettige oder verzweigte Alkylgruppe bedeutet, die bis zu 6 Kohlenstoffatomen enthält, die ggfs. mit einem oder mehreren Halogenatomen substituiert ist.

10. Verfahren nach Anspruch 1, worin bedeuten:
R eine Gruppe -CO₂R³ oder -COR⁵;
R¹ eine geradkettige oder verzweigte Alkylgruppe, die bis zu 6 Kohlenstoffatome enthält, oder eine Cycloalkylgruppe, die 3 oder 4 Kohlenstoffatome enthält und die ggfs. mit einer oder mehrerer Gruppen R⁵ stubstituiert ist;
R² ein Halogenatom oder
eine Alkylgruppe, die bis zu 6 Kohlenstoffatome enthält, die mit -OR⁵ substituiert ist, oder
eine Gruppe R⁵, -NO², -CO₂R³, -SR⁵, -SOR⁵, -SO₂R⁵, -OR⁵ oder eine Gruppe -O(CH₂)q-OR⁵;
R³ eine geradkettige oder verzweigte Alkylgruppe, die bis zu 6 Kohlenstoffatome enthält;
R³¹ Wasserstoff;
R⁴ Wasserstoff;
R⁵ eine geradkettige oder verzweigte Alkylgruppe, die bis zu 5 Kohlenstoffatome enthält, die ggfs. mit einem oder mehreren Halogenatomen substitutiert ist;
eine der Gruppen R² am Phenylring in ortho-Stellung vorliegt, und
n 2 oder 3.

11. Verfahren nach Anspruch 1 zur Herstellung von:
3-Methyl-4-(2-nitro-4-trifluoromethylbenzoyl)-isoxazol;
3-(1-Methylethyl)-4-(2-nitro-4-trifluormethylbenzoyl)-isoxazol;
Ethyl-4-(2-nitro-4-trifluormethylbenzoyl)-isoxazol-3-carboxylat;
3-Brom-4-(2-nitro-4-trifluormethylbenzoyl)-isoxazol;
4-(2-Nitro-4-trifluormethylbenzoyl)-3-trifluormethylisoxazol;
5-Cyclopropyl-3-methyl-4-(2-nitro-4-trifluormethylbenzoyl)-isoxazol;
Ethyl-5-methyl-4-(2-nitro-4-trifluormethylbenzoyl)-isoxazol-3-carboxylat;
5-Methyl-4-(2-nitro-4-trifluormethylbenzoyl)-isoxazol-3-carboxamid;
Ethyl-5-cyclopropyl-4-(2-nitro-4-trifluormethylbenzoyl)ixoxazol-3-carboxylat;
3-Cyano-5-methyl-4-(2-nitro-4-trifluormethylbenzoyl)-isoxazol oder
Ethyl-4-(2-chlor-4-methylsulfonylbenzoyl)-5-cyclopropylisoxazol-3-carboxylat.

12. Verfahren nach Anspruch 1 zur Herstellung von:
Ethyl-5-cyclopropyl-4-(4-methylsulfonyl-2-trifluormethylbenzoyl)-isoxazol-3-carboxylat;
Methyl-4-(2-chlor-4-methylsulfonylbenzoyl)-5-cyclopropylisoxazol-3-carboxylat;
4-(2-chlor-4-methylsuphonylbenzoyl)-5-cyclopropylisoxazol-3-carboxamid;
Ethyl-5-cyclopropyl-4-(2-methylsulfonylbenzoyl)ixoxazol-3-carboxylat;
Methyl-5-cyclopropyl-4-methylsulfonyl-2-trifluormethylbenzoyl)-isoxazol-3-carboxylat;
Ethyl-5-cyclopropyl-4-(2-nitro-4-methylsulfonylbenzoyl)-isoxazol-3-carboxylat;
Ethyl-5-cyclopropyl-4-(2,3-dichlor-4-methylsulfonylbenzoyl)-isocazol-3-carboxylat;
Ethyl-4-(2-chlor-3-methoxy-4-methylsulfonylbenzoyl)-5-cyclopropylisoxazol-3-carboxylat;
4-(2-chlor-4-methylsulfonylbenzoyl)-3-cyano-5-cyclopropylisoxazol;
Ethyl-4-(4-chlor-2-methylsulfonylbenzoyl)-5-cyclopropylisoxazol-3-carboxylat;
Ethyl-4-(4-chlor-2-nitrobenzoyl)-5-cyclopropylisoxazol-3-carboxylat;
Ethyl-4-(4-chlor-2-trifluormethylbenzoyl)-5-cyclopropylisoxazol-3-carboxylat;
Ethyl-5-cyclopropyl-4-(2-methylsulfenyl-4-trifluormethylbenzoyl)-isoxazol-3-carboxylat;
Ethyl-5-cyclopropyl-4-(4-methylsulfenyl-2-trifluormethylbenzoyl)-isoxazol-3-carboxylat;
Ethyl-5-cyclopropyl-4-(2-methylsulfinyl-4-trifluormethylbenzoyl)-isoxazol-3-carboxylat;
Ethyl-5-cyclopropyl-4-(4-methylsulfinyl-2-trifluormethylbenzoyl)-isoxazol-3-carboxylat;
Ethyl-5-cyclopropyl-4-(2-methylsulfonyl-4-trifluormethylbenzoyl)-isoxazol-3-carboxylat;
Ethyl-5-cyclopropyl-4-(2-fluor-4-methylsulfonylbenzoyl)isoxazol-3-carboxylat;
Ethyl-4-(2-chlor-4-ethylsulfonylbenzoyl)-5-cyclopropylisoxazol-3-carboxylat;
Ethyl-4-(4-brom-2-methylsulfonylbenzoyl)-5-cyclopropylisoxazol-3-carboxylat;
Ethyl-4-(2-brom-4-methylsulfonylbenzoyl)-5-cyclopropylisoxazol-3-carboxylat;
Ethyl-4-[2-chlor-4-methylsulfenylbenzoyl]-5-cyclopropylisoxazol-3-carboxylat;
Ethyl-4-(2-chlor-4-methylsulfinylbenzoyl)-5-cyclopropylisoxazol-3-carboxylat;
3-Acetyl-4-(2-chlor-4-methylsulfonylbenzoyl)-5-cyclopropylisoxazol;
Isopropyl-4-(2-chlor-4-methylsulfonylbenzoyl)-5-cyclopropylisoxazol-3-carboxylat;
Methyl-4-(4-chloro-2-methylsulfonylbenzoyl)-5-cyclopropylisoxazol-3-carboxylat;
Methyl-5-cyclopropyl-4-(2-methylsulfonyl-4-trifluormethylbenzoyl)-isoxazol-3-carboxylat;
Methyl-4-[4-chlor-2-methylsulfenylbenzoyl]-5-cyclopropylisoxazol-3-carboxylat oder
Methyl-4-(4-chlor-2-methylsulfinylbenzoyl)-5-cyclopropylixoxazol-3-carboxylat.

13. Verfahren nach Anspruch 1 zur Herstellung von: Ethyl-5-cyclopropyl-4-(2-methylsulfonyl-4-trifluormethylbenzoyl)-isoxazol-3-carboxylat.

14. Verfahren zur Herstellung einer herbiziden Zusammensetzung, das die Formulierung einer herbizid wirksamen Menge eines 4-Benzoylisoxazolderivats der Formel I nach Anspruch 1 als Wirkstoff umfaßt,
worin bedeuten:
R eine geradkettige oder verzweigte Alkyl-, Alkenyl- oder Alkinylgruppe, die bis zu 6 Kohlenstoffatome enthält und die ggfs. mit einem oder mehreren Halogenatomen substituiert ist, oder
eine Cycloalkylgruppe, die 3 bis 6 Kohlenstoffatome enthält, die ggfs. mit einem oder mehreren Substitutenten substituiert ist, die unter den Gruppen R⁵, einem oder mehreren Halogenatomen und unter einer Gruppe -CO₂R₃ ausgewählt sind, oder
eine Gruppe, die unter -CO₂R³ , -COR⁵, Cyano, Nitro und -CONR³¹R⁴ ausgewählt ist, oder
ein Halogenatom;
R¹ ein Wasserstoffatom oder eine geradkettige oder verzweigte Alkyl-, Alkenyl- oder Alkinylgruppe, die bis zu 6 Kohlenstoffatome enthält, die ggfs. mit einem oder mehreren Halogenatomen substituiert ist, oder
eine Cycloalkylgruppe, die 3 bis 6 Kohlenstoffatome enthält und die ggfs. mit einem oder mehreren Substitutenten substituiert ist, die unter den Gruppen R⁵, einem oder mehreren Halogenatomen und unter einer Gruppe -CO₂R₃ ausgewählt sind;
R² ein Halogenatom oder
eine Gruppe, die unter R⁵, -SR⁵, -SOR⁵, SO₂R⁵, -SO₂NR³¹R⁴, -CO₂R³, -COR⁵, -CONR³¹R⁴, -CSNR³¹R⁴, -OR⁵, einer Nitrogruppe, einer Cyanogruppe, einer Gruppe -O(CH₂)q-OR⁵ oder einer geradkettigen oder verzweigten Alkylgruppe ausgewählt ist, die bis zu 6 Kohlenstoffatome enthält und mit OR⁵ substituiert ist;
worin R³, R³¹ und R⁴ gleich oder voneinander verschieden sind und worin bedeuten:
ein Wasserstoffatom oder
eine geradkettige oder verzweigte Alkylgruppe, die bis zu 6 Kohlenstoffatome enthält und die ggfs. mit einem oder mehreren Halogenatomen substituiert ist;
R⁵ eine geradkettige oder verzweigte Alkylgruppe, die bis zu 6 Kohlenstoffatome enthält und die ggfs. mit einem oder mehreren Halogenatomen substituiert ist;
n eine ganze Zahl von 1 bis 5;
q eine ganze Zahl von 1 bis 3
mit der Maßgabe, daß R und R¹ nicht gleichzeitig eine Methylgruppe bedeuten, oder
ein für die Landwirtschaft akzeptables Salz davon.

15. Verfahren zur Herstellung einer herbiziden Zusammensetzung, das die Formulierung einer herbizid wirksamen Menge einer Verbindung nach einem der Ansprüche 1 bis 13 als Wirkstoff in Verbindung mit einem von der Herbizidwirkung akzeptablen Verdünnungsmittel oder einem Träger und/oder einem für Herbizide akzeptablen grenzflächenaktiven Mittel umfaßt.

16. Verfahren nach Anspruch 14 oder 15, wobei die herbizide Zusammensetzung 0,05 bis 90 Gew.-% Wirkstoff enthält.

17. Verfahren nach einem der Ansprüche 14 bis 16, wobei die herbizide Zusammensetzung in flüssiger Form vorliegt und 0,05 bis 25 Gew.-% grenzflächenaktives Mittel enthält.

18. Verfahren nach einem der Ansprüche 14 bis 16, wobei die herbizide Zusammensetzung in Form eines wäßrigen Suspensionskonzentrats oder eines Spritzpulvers oder eines wasserlöslichen oder in Wasser dispergierbaren Pulvers oder eines flüssigen wasserlöslichen oder in Wasser dispergierbaren Konzentrats oder einer flüssigen emulgierbaren Suspension oder eines Granulats vorliegt.

19. Verfahren nach Anspruch 18, wobei die herbizide Zusammensetzung in Form
einer wäßrigen Suspension, die 10 bis 70 Gew.-% Wirkstoff enthält, oder
eines Spritzpulvers, das 10 bis 90 Gew.-% Wirkstoff enthält, oder
eines in Wasser dispergierbaren oder wasserlöslichen Pulvers, das 10 bis 90 Gew.-% Wirkstoff enthält, oder
eines flüssigen wasserlöslichen Konzentrats, das 5 bis 50 Gew.-% Wirkstoff enthält, oder
eines flüssigen emulgierbaren Suspensionskonzentrats, das 10 bis 70 Gew.-% Wirkstoff enthält, oder
eines Granulats vorliegt, das 1 bis 90 Gew.-% Wirkstoff enthält.

20. Verfahren nach Anspruch 18 oder 19, wobei die herbizide Zusammensetzung in Form einer wäßrigen Suspension, die 2 bis 10 Gew.-% grenzflächenaktives Mittel enthält, oder eines Spritzpulvers, das 2 bis 10 Gew.-% grenzflächenaktives Mittel enthält, oder eines flüssigen emulgierbaren Suspensionskonzentrats, das 5 bis 15 Gew.-% grenzflächenaktives Mittel enthält, oder eines flüssigen wasserlöslichen Konzentrats, das 5 bis 25 Gew.-% grenzflächenaktives Mittel enthält, oder eines Granulats vorliegt, das 0,5 bis 7 Gew.-% grenzflächenaktives Mittel enthält.

21. Verfahren zur lokalen Kontrolle des Wachstums von Unkräutern, das das Ausbringen einer herbizid wirksamen Menge eines 4-Benzoylisoxazolderivats nach einem der Ansprüche 1 bis 14 oder eines in der Landwirtschaft akzeptablen Salzes davon am betreffenden Ort umfaßt.

22. Verfahren nach Anspruch 21, wobei der Ort eine genutzte oder zu nutzende Anbaufläche ist und die Ausbringmenge 0,01 bis 4,0 kg Wirkstoff pro Hektar beträgt.

23. Verfahren nach Anspruch 21, wobei der Ort keine Anbaufläche ist und die Ausbringmenge 1,0 bis 20,0 kg Wirkstoff pro Hektar beträgt.

24. 4-Benzoylixoxazolderivate nach einem der Ansprüche 1 bis 14.

25. Herbizide Zusammensetzung nach einem der Ansprüche 14 bis 20.

## Revendications (Revendications pour l'(les) Etat(s) contractant(s) suivant(s): AT, BE, CH, LI, DE, DK, FR, GB, GR, IT, LU, NL, SE)

1. Un dérivé du 4-benzoyl isoxazole de formule générale I 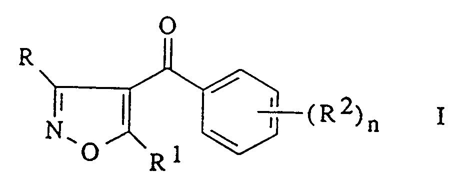 dans laquelle :
R représente:,
un radical alkyle, alkényle ou alkynyle linéaire ou ramifié, contenant de 1 à 6 atomes de carbone, éventuellement substitué par un ou plusieurs atomes d'halogènes ; ou
un radical cycloalkye,contenant de 3 à 6 atomes de carbone, éventuellement substitué par un ou plusieurs substituants choisis parmi les radicaux R⁵, un ou plusieurs atomes d'halogènes et un radical CO₂R₃ ; ou
un radical choisi parmi les radicaux suivants: CO₂R³, -COR⁵, cyano, nitro, -CONR³¹R⁴, ou
un atome d'halogène ;
R¹ représente :
Un atome d'hydrogène, ou
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle,contenant de 3 à 6 atomes de carbone, éventuellement substitué par un ou plusieurs substituants choisis parmi les radicaux R⁵ et un ou plusieurs atomes d'halogènes ;
R² représente :
un atome d'halogène, ou
Un radical choisi parmi les radicaux suivants : R⁵, -SR⁵, SOR⁵ SO₂R⁵, -SO₂NR³¹R⁴, -CO₂R³, -COR⁵, CONR³¹R⁴, CSNR³¹R⁴, -OR⁵, un radical nitro, un radical cyano, un radical -O(CH₂)_{q}-OR⁵, ou un radical alkyle, linéaire ou ramifié, contenant de 1 à 6 atomes de carbone, et qui est substitué par un radical OR⁵;
R³, R³¹ et R⁴, qui peuvent être identiques ou différents, représentent chacun
un atome d'hydrogène, ou
un radical alkyle, alkényle ou alkynyle linéaire ou ramifié, contenant de 1 à 6 atomes de carbone, éventuellement substitué par un ou plusieurs atomes d'halogènes ; ou
R⁵ représente :
un radical alkyle, alkényle ou alkynyle linéaire ou ramifié, contenant de 1 à 6 atomes de carbone, éventuellement substitué par un ou plusieurs atomes d'halogènes ;
n représente un nombre entier pouvant prendre toutes les valeurs de 1 à 5 ;
q représente un nombre entier pouvant prendre toutes les valeurs de 1 à 3 ;
à condition que R et R¹ ne représentent pas simultanément un radical méthyle ;
et les sels de ces composés acceptables pour des usages agricoles.

2. Un composé selon la revendication 1 dans lequel au moins un des radicaux R² est en position ortho adjacente au radical carbonyle reliant les cycles phényle et isoxazolyle.

3. Un composé selon la revendication 1 ou 2 dans laquelle R représente :
un radical alkyle, linéaire ou ramifié, contenant de 1 à 6 atomes de carbone, éventuellement substitué par un ou plusieurs atomes d'halogènes;
un radical cycloalkyle, contenant de 3 à 6 atomes de carbone, ou
un radical choisi parmi les radicaux suivants: CO₂R³, -COR⁵,
ou un atome d'halogène.

4. Un composé selon la revendication 1, 2 ou 3 dans laquelle :
R¹ représente :
un radical alkyle, linéaire ou ramifié, contenant de 1 à 6 atomes de carbone,
un radical cycloalkyle,contenant de 3 à 4 atomes de carbone, éventuellement substitué par un ou plusieurs radicaux R^{5.}

5. Un composé selon la revendication 4 dans laquelle R¹ est
choisi parmi les radicaux méthyle, isopropyle, cyclopropyle ou 1-méthylcyclopropyle.

6. Un composé selon l'une quelconque des revendications précédentes dans la quelle R² représente
un atome d'halogène, ou
un radical alkyle contenant de 1 à 6 atomes de carbone, substitué par un radical -OR⁵, ou
un radical choisi parmi les radicaux suivants R⁵, nitro, CO₂R³, -SR⁵, SO₂R⁵, SOR⁵ et -OR⁵;

7. Un composé selon l'une quelconque des revendications précédentes dans la quelle R³ représente :
un atome d'hydrogène, ou
un radical alkyle linéaire ou ramifié, contenant de 1 à 6 atomes de carbone.

8. Un composé selon l'une quelconque des revendications précédentes dans la quelle R⁴ représente un atome d'hydrogène.

9. Un composé selon l'une quelconque des revendications précédentes dans la quelle R⁵ représente un radical alkyle linéaire ou ramifié, contenant de 1 à 6 atomes de carbone éventuellement substitué par un ou plusieurs atomes d'halogène.

10. Un composé selon la revendication 1 dans laquelle :
R représente un radical -CO₂R³ ou -COR⁵;
R¹ représente :
un radical alkyle, linéaire ou ramifié, contenant de 1 à 6 atomes de carbone,
un radical cycloalkyle,contenant de 3 à 4 atomes de carbone, éventuellement substitué par un ou plusieurs substituants choisis parmi un ou plusieurs radicaux R⁵,
R² représente :
un atome d'halogène ; ou
un radical alkyle contenant de 1 à 6 atomes de carbone, substitué par un radical -OR⁵ ou
un radical choisi parmi les radicaux suivants R⁵, un radical nitro, CO₂R³, -SR⁵, SO₂R⁵, SOR⁵, -OR⁵ ,ou
un radical -O(CH₂)_{q}-OR⁵;
R³ représente un radical alkyle, linéaire ou ramifié, contenant de 1 à 6 atomes de carbone ;
R³¹ représente un atome d'hydrogène
R⁴ représente un atome d'hydrogène,
R⁵ représente un radical alkyle, linéaire ou ramifié, contenant de 1 à 6 atomes de carbone, éventuellement substitué par un ou plusieurs atomes d'halogènes ;
un des radicaux R² est en position ortho sur le cycle phényle ;
n représente 2 ou 3.

11. Un composé selon la revendication 1, qui est le :
3-Méthyl-4-(2-nitro-4-trifluorométhylbenzoyl)-isoxazole ;
3-(1-Méthyléthyl)-4-(2-nitro-4-trifluorométhylbenzoyl)-isoxazole ;
3-Ethyl-4-(2-nitro-4-trifluorométhylbenzoyl)-isoxazole-3-carboxylate ;
3-Bromo-4-(2-nitro-4-trifluorométhylbenzoyl)-isoxazole ;
4-(2-nitro-4-trifluorométhylbenzoyl)-3-trifluorométhylisoxazole ;
5-Cyclopropyl-3-Méthyl-4-(2-nitro-4-trifluorométhylbenzoyl)-isoxazole ;
5-Méthyl-4-(2-nitro-4-trifluorométhylbenzoyl)-isoxazole-3-carboxylate d'éthyle ;
5-Méthyl-4-(2-nitro-4-trifluorométhylbenzoyl)-isoxazole-3-carboxamide ;
5-Cyclopropyl-4-(2-nitro-4-trifluorométhylbenzoyl)-isoxazole-3-carboxylate d'éthyle ;
3-Cyano-5-Méthyl-4-(2-nitro-4-trifluorométhylbenzoyl)-isoxazole ;
4-(2-chloro-4-méthylsulfonylbenzoyl)-5-cyclopropylisoxazole-3-carboxylate d'éthyle ;

12. Un composé selon la revendication 1, qui est le :
5-Cyclopropyl-4-(4-méthylsulfonyl-2-trifluorométhylbenzoyl)-isoxazole-3-carboxylate d'éthyle ;
4-(2-chloro-4-méthylsulfonylbenzoyl)-5-cyclopropylisoxazole-3-carboxylate de méthyle ;
4-(2-chloro-4-méthylsulfonylbenzoyl)-5-cyclopropylisoxazole-3-carboxamide
5-Cyclopropyl-4-(2-méthylsulfonylbenzoyl)-isoxazole-3-carboxyate d'éthyle
5-Cyclopropyl-4-(4-méthylsulfonyl-2-trifluorométhylbenzoyl)-isoxazole-3-carboxylate de méthyle ;
5-Cyclopropyl-4-(2-nitro-4-méthylsulfonylbenzoyl)-isoxazole-3-carboxylate d'éthyle ;
5-Cyclopropyl-4-(2,3-dichloro-4-méthylsulfonylbenzoyl)-isoxazole-3-carboxylate d'éthyle ;
4-(2-Chloro-3-méthoxy-4-méthylsulfonylbenzoyl)-5-cyclopropylisoxazole-3-carboxylate d'éthyle ;
4-(2-Chloro-4-méthylsulfonylbenzoyl)-3-cyano-5-cyclopropylisoxazole ;
4-(4-Chloro-2-méthylsulfonylbenzoyl)-5-cyclopropylisoxazole-3-carboxylate d'éthyle ;
4-(4-Chloro-2-nitrobenzoyl)-5-cyclopropylisoxazole-3-carboxylate d'éthyle
4-(4-chloro-2-trifluorométhylbenzoyl)-5-cyclopropylisoxazole-3-carboxylate d'éthyle ;
5-Cyclopropyl-4-(2-méthysulfényl-4-trifluorométhylbenzoyl)-isoxazole-3-carboxylate d'éthyle ;
5-Cyclopropyl-4-(4-méthylsulfényl-2-trifluorométhylbenzoyl)-isoxazole-3-carboxylate d'éthyle ;
5-Cyclopropyl-4-(2-méthylsulfinyl-4-trifluorométhylbenzoyl)-isoxazole-3-carboxylate d'éthyle ;
5-Cyclopropyl-4-(4-méthylsulfinyl-2-trifluorométhylbenzoyl)-isoxazole-3-carboxylate d'éthyle ;
5-Cyclopropyl-4-(2-méthylsulfonyl-4-trifluorométhybenzoyl)-isoxazole-3-carboxylate d'éthyle ;
5-Cyclopropyl-4-(2-fluoro-4-méthylsulfonylbenzoyl)-isoxazole-3-carboxylate d'éthyle ;
4-(2-Chloro-4-éthylsulfonylbenzoyl)-5-cyclopropylisoxazole-3-carboxylate d'éthyle ;
4-(4-Bromo-2-méthylsulfonylbenzoyl)-5-cyclopropylisoxazole-3-carboxylate d'éthyle ;
4-(2-Bromo-4-méthylsulfonylbenzoyl)-5-cyclopropylisoxazole-3-carboxylate d'éthyle ;
4-(2-Choro-4-méthylsulfénylbenzoyl)-5-cyclopropylisoxazole-3-carboxylate d'éthyle ;
4-(2-Choro-4-méthylsulfinylbenzoyl)-5-cyclopropylisoxazole-3-carboxylate d'éthyle ;
3-Acétyl-4-(2-Chloro-4-méthylsulfonylbenzoyl)-5-cyclopropylisoxazole
4-(2-Chloro-4-méthylsulfonylbenzoyl)-5-cyclopropylisoxazole-3-carboxylate d'isopropyle ;
4-(4-Chloro-2-méthylsulfonylbenzoyl)-5-cyclopropylisoxazole-3-carboxylate de méthyle ;
5-Cyclopropyl-4-(2-méthylsulfonyl-4-trifluorométhylbenzoyl)-isoxazole-3-carboxylate de méthyle ;
4-(4-Chloro-2-méthylsulfénylbenzoyl)-5-cyclopropylisoxazole-3-carboxylate de méthyle ;
4-(4-Chloro-2-méthylsulfinylbenzoyl)-5-cyclopropylisoxazole-3-carboxylate de méthyle ;

13. Un composé selon la revendication 1 qui est le :
5-Cyclopropyl-4-(2-méthylsulfonyl-4-trifluorométhylbenzoyl)-isoxazole-3-carboxylate d'éthyle.

14. Un procédé de préparation d'un composé selon la revendication 1 qui comprend les étapes suivantes :
a) action d'un composé de formule générale II 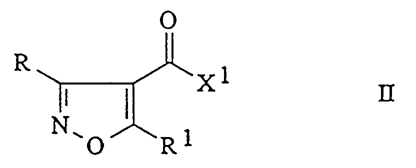 dans laquelle R et R¹ ont la définition donnée dans la revendication 1, et X¹ représente un atome d'halogène, sur un composé de formule générale III 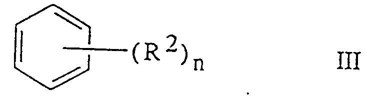 dans laquelle R² et n ont la définition donnée dans la revendication 1, en présence d'un catalyseur acide de Lewis,
b) action d'un composé de formule générale IV, 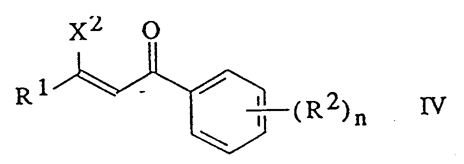 dans laquelle R et R¹ ont la définition donnée dans la revendication 1, et X² représente un un radical de formule -N(R⁷)₂ ou -SR⁷, dans laquelle R⁷ représente un radical alkyle, linéaire ou ramifié, contenant de 1 à 4 atomes de carbone, sur un composé de formule
RC(X¹)=N-OH
dans laquelle R et X¹ ont la définition donnée dans la revendication 1, en présence d'une base organique, dans un solvant inerte ;
c) action d'un composé de formule générale VI 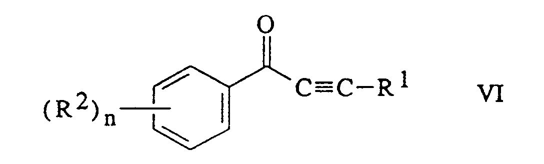 dans laquelle R¹, R² et n ont la définition donnée dans la revendication 1, sur un composé de formule
RC(X¹)=N-OH
dans laquelle R a la définition donnée dans la revendication 1 et X¹ représente un atome d'halogène, en présence d'une base organique ou d'un catalyseur dans un solvant inerte ;
d) oxydation du composé de formule générale VII 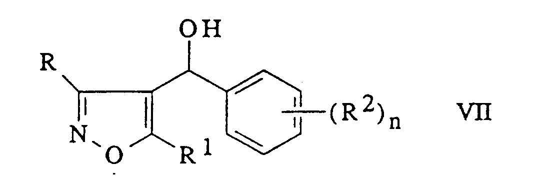 dans laquelle R, R¹, R² et n ont la définition donnée dans la revendication 1, pour transformer le radical hydroxy en un radical cétone,
e) métallation d'un composé de formule générale VIII 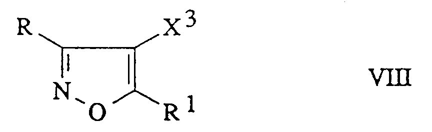 dans laquelle R et R¹ ont la définition donnée dans la revendication 1, et X³ représente le brome ou l'iode, suivie par un traitement par un chlorure de benzoyle de formule générale IX 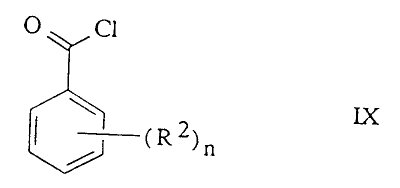 dans laquelle R² et n ont la définition donnée ci-dessus.
f) action d'un sel d'un composé de formule générale X 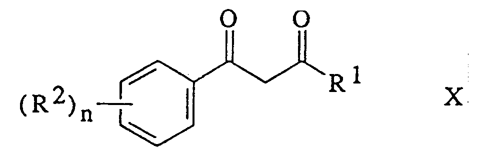 dans laquelle R¹, R² et n ont la définition donnée ci-dessus, sur un composé de formule générale :
RC(X¹)=N-OH
dans laquelle R a la définition donnée dans la revendication 1 et X¹ représente un atome d'halogène ;
et transformation éventuelle du dérivé du 4-benzoyl isoxazole de formule générale I ainsi obtenu en un sel, acceptable pour des applications agricoles, du dit composé.

15. Une composition herbicide contenant comme matière active une quantité herbicide efficace d'un dérivé du 4-benzoyl isoxazole de formule générale 1 tel que défini dans la revendication 1 dans laquelle :
R représente:,
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle,contenant de 3 à 6 atomes de carbone, éventuellement substitué par un ou plusieurs substituants choisis parmi les radicaux R⁵, un ou plusieurs atomes d'halogènes et un radical CO₂R₃ ; ou
un radical choisi parmi les radicaux suivants: CO₂R³, -COR⁵, cyano, nitro, -CONR³¹R⁴, ou
un atome d'halogène ;
R¹ représente :
Un atome d'hydrogène, ou
un radical alkyle, alkényle ou alkynyle linéaire ou ramifié contenant de 1 à 6 atomes de carbone, éventuellement substitué par un ou plusieurs atomes d'halogènes ; ou
un radical cycloalkyle,contenant de 3 à 6 atomes de carbone, éventuellement substitué par un ou plusieurs substituants choisis parmi les radicaux R⁵ et un ou plusieurs atomes d'halogènes ;
R² représente :
un atome d'halogène, ou
Un radical choisi parmi les radicaux suivants : R⁵, -SR⁵, SOR⁵ SO₂R⁵, -SO₂NR³¹R⁴, -CO₂R³, -COR⁵, CONR³¹R⁴, CSNR³¹R⁴, -OR⁵, un radical nitro, un radical cyano, un radical -O(CH₂)_{q}-OR⁵, ou un radical alkyle, linéaire ou ramifié, contenant de 1 à 6 atomes de carbone, et qui est substitué par un radical OR⁵ ;
R³, R³¹ et R⁴, qui peuvent être identiques ou différents, représentent chacun
un atome d'hydrogène, ou
un radical alkyle, alkényle ou alkynyle linéaire ou ramifié, contenant de 1 à 6 atomes de carbone, éventuellement substitué par un ou plusieurs atomes d'halogènes ; ou
R⁵ représente :
un radical alkyle, alkényle ou alkynyle linéaire ou ramifié, contenant de 1 à 6 atomes de carbone, éventuellement substitué par un ou plusieurs atomes d'halogènes ;
n représente un nombre entier pouvant prendre toutes les valeurs de 1 à 5 ;
q représente un nombre entier pouvant prendre toutes les valeurs de 1 à 3 ;
à condition que R et R¹ ne représentent pas simultanément un radical méthyle ;
et les sels de ces composés acceptables pour des usages agricoles, associé avec des diluants ou des charges acceptables pour des applications herbicides et/ou des agents tensio-actifs acceptables pour des applications herbicides.

16. Une composition herbicide contenant comme matière active une quantité herbicide efficace d'un composé selon l'une quelconque des revendications 1 à 13 associé avec des diluants ou des charges acceptables pour des applications herbicides et/ou des agents tensio-actifs acceptables pour des applications herbicides.

17. Une composition herbicide selon la revendication 15 ou 16 qui contient de 0,05 à 90% en poids de la matière active.

18. Une composition herbicide selon l'une quelconque des revendications 15 à 17 qui se présente sous forme liquide et contient de 0,05 à 25% en poids d'un agent tensioactif.

19. Une composition herbicide selon l'une quelconque des revendications 15 à 17 qui se présente sous forme d'une suspension aqueuse concentrée ou d'une poudre mouillable ou d'une poudre soluble dans l'eau ou d'une poudre dispersible dans l'eau, ou d'un concentré liquide soluble dans l'eau ou dispersible dans l'eau ou d'une suspension liquide émulsionnable ou d'un granulé.

20. Une composition herbicide selon la revendication 19 qui se présente sous forme d'une suspension aqueuse contenant de 10 à 70% de matière active, ou d'une poudre mouillable contenant de 10 à 90% de matière active, ou d'une poudre soluble dans l'eau ou d'une poudre dispersible dans l'eau contenant de 10 à 90% de matière active, ou d'un concentré liquide soluble dans l'eau ou dispersible dans l'eau contenant de 5 à 50% de matière active, ou d'une suspension liquide concentrée émulsionnable contenant de 10 à 70% de matière active ou de granulés contenant de 1 à 90% de matière active, les pourcentages étant donnés en poids.

21. Une composition herbicide selon la revendication 19 ou 20 dans laquelle la composition se présente sous forme d'une suspension aqueuse contenant de 2 à 10% d'un agent tensioactif, ou d'une poudre mouillable contenant de 2 à 10% d'un agent tensioactif, ou d'une suspension liquide concentrée émulsionnable contenant de 5 à 15% d'un agent tensioactif, ou d'un concentré liquide soluble dans l'eau contenant de 5 à 25% d'un agent tensioactif, ou de granulés contenant de 0,5 à 7% d'un agent tensioactif, les pourcentages étant donnés en poids.

22. Un procédé de contrôle de la croissance des mauvaises herbes dans un lieu consistant à appliquer sur le dit-lieu une quantité herbicide efficace d'un dérivé du 4-benzoyl isoxazole selon l'une quelconque des revendications 1 à 13, ou selon la revendication 15, ou d'un sel acceptable pour des applications agricoles, de ces composés.

23. Un procédé selon la revendication 22 dans lequel le lieu est une zone utilisée ou destinée à être utilisée pour y faire pousser des cultures, et où la dose d'emploi utilisée est comprise entre 0,01 et 4 kg de matière active par hectare.

24. Un procédé selon la revendication 22 dans lequel le lieu est une zone de non-culture et où la dose d'application est comprise entre 1,0 et 20 kg de matière active par hectare.

## Revendications (Revendications pour l'(les) Etat(s) contractant(s) suivant(s): ES)

1. Un procédé de préparation d'un dérivé du 4-benzoyl isoxazole de formule générale I : 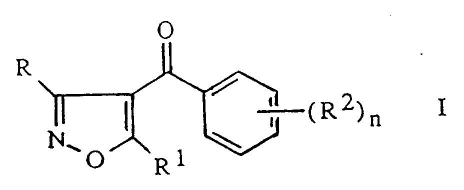 dans laquelle :
R représente:,
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle,contenant de 3 à 6 atomes de carbone, éventuellement substitué par un ou plusieurs substituants choisis parmi les radicaux R⁵, un ou plusieurs atomes d'halogènes et un radical CO₂R₃ ; ou
un radical choisi parmi les radicaux suivants: CO₂R³, -COR⁵, cyano, nitro, -CONR³¹R⁴ ; ou
un atome d'halogène ;
R¹ représente :
un atome d'hydrogène, ou
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle,contenant de 3 à 6 atomes de carbone, éventuellement substitué par un ou plusieurs substituants choisis parmi les radicaux R⁵ et un ou plusieurs atomes d'halogènes ;
R² représente :
un atome d'halogène, ou
Un radical choisi parmi les radicaux suivants : R⁵, -SR⁵, SOR⁵ SO₂R⁵, -SO₂NR³¹R⁴, -CO₂R³, -COR⁵, CONR³¹R⁴, CSNR³¹R⁴, -OR⁵, un radical nitro, un radical cyano, un radical -O(CH₂)_{q}-OR⁵, ou un radical alkyle, linéaire ou ramifié, contenant de 1 à 6 atomes de carbone, et qui est substitué par un radical OR⁵ ;
R³, R³¹ et R⁴, qui peuvent être identiques ou différents, représentent chacun
un atome d'hydrogène, ou
un radical alkyle, alkényle ou alkynyle linéaire ou ramifié, contenant de 1 à 6 atomes de carbone, éventuellement substitué par un ou plusieurs atomes d'halogènes ; ou
R⁵ représente :
un radical alkyle, alkényle ou alkynyle linéaire ou ramifié, contenant de 1 à 6 atomes de carbone, éventuellement substitué par un ou plusieurs atomes d'halogènes ;
n représente un nombre entier pouvant prendre toutes les valeurs de 1 à 5 ;
q représente un nombre entier pouvant prendre toutes les valeurs de 1 à 3 ;
à condition que R et R¹ ne représentent pas simultanément un radical méthyle ;
et les sels de ces composés acceptables pour des usages agricoles.
le-dit procédé comprenant les étapes suivantes :
a) action d'un composé de formule générale II 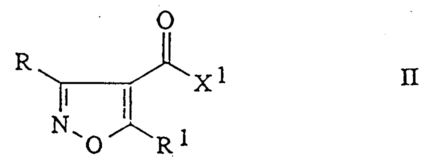 dans laquelle R et R¹ ont la définition donnée ci-dessus , et X¹ représente un atome d'halogène, sur un composé de formule générale III 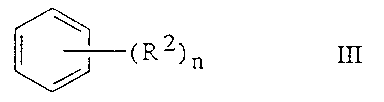 dans laquelle R² et n ont la définition donnée ci-dessus , en présence d'un catalyseur acide de Lewis,
b) action d'un composé de formule générale IV, 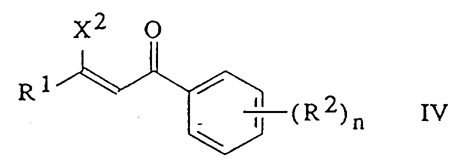 dans laquelle R et R¹ ont la définition donnée ci-dessus, et X² représente un un radical de formule -N(R⁷)₂ ou -SR⁷, dans laquelle R⁷ représente un radical alkyle, linéaire ou ramifié, contenant de 1 à 4 atomes de carbone, sur un composé de formule
RC(X¹)=N-OH
dans laquelle R et X¹ ont la définition donnée ci-dessus, en présence d'une base organique, dans un solvant inerte ;
c) action d'un composé de formule générale VI 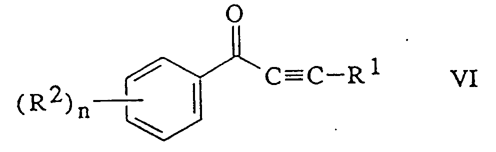 dans laquelle R¹, R² et n ont la définition donnée ci-dessus, sur un composé de formule
RC(X¹)=N-OH
dans laquelle R a la définition donnée ci-dessus, et X¹ représente un atome d'halogène, en présence d'une base organique ou d'un catalyseur dans un solvant inerte ;
d) oxydation du composé de formule générale VII 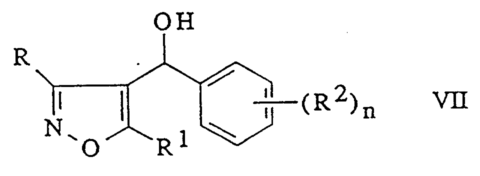 dans laquelle R, R¹, R² et n ont la définition donnée ci-dessus, pour transformer le radical hydroxy en un radical cétone,
e) métallation d'un composé de formule générale VIII 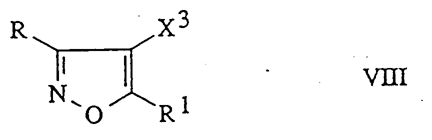 dans laquelle R et R¹ ont la définition donnée ci-dessus et X³ représente le brome ou l'iode, suivie par un traitement par un chlorure de benzoyle de formule générale IX 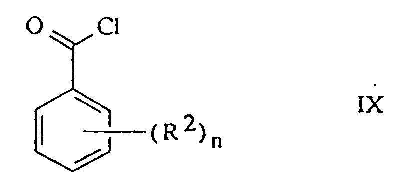 dans laquelle R² et n ont la définition donnée ci-dessus.
f) action d'un sel d'un composé de formule générale X 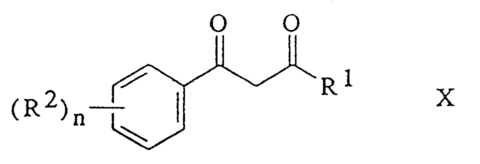 dans laquelle R¹, R² et n ont la définition donnée ci-dessus, sur un composé de formule générale :
RC(X¹)=N-OH
dans laquelle R a la définition donnée ci-dessus, et X¹ représente un atome d'halogène ;
et transformation éventuelle du dérivé du 4-benzoyl isoxazole de formule générale I ainsi obtenu en un sel, acceptable pour des applications agricoles, du dit composé.

2. Un procédé selon la revendication 1 pour la préparation d'un composé dans lequel au moins un des radicaux R² est en position ortho adjacente au radical carbonyle reliant les cycles phényle et isoxazolyle.

3. Un procédé selon la revendication 1 ou 2 dans laquelle R représente :
un radical alkyle, linéaire ou ramifié, contenant de 1 à 6 atomes de carbone, éventuellement substitué par un ou plusieurs atomes d'halogènes;
un radical cycloalkyle, contenant de 3 à 6 atomes de carbone, ou
un radical choisi parmi les radicaux suivants: CO₂R³, -COR⁵,
ou un atome d'halogène.

4. Un procédé selon la revendication 1, 2 ou 3 dans laquelle :
R¹ représente :
un radical alkyle, linéaire ou ramifié, contenant de 1 à 6 atomes de carbone,
un radical cycloalkyle,contenant de 3 à 4 atomes de carbone, éventuellement substitué par un ou plusieurs radicaux R^{5.}

5. Un procédé selon la revendication 4 dans laquelle R¹ est
choisi parmi les radicaux méthyle, isopropyle, cyclopropyle ou 1 -méthylcyclopropyle.

6. Un procédé selon l'une quelconque des revendications précédentes dans la quelle R² représente
un atome d'halogène, ou
un radical alkyle contenant de 1 à 6 atomes de carbone, substitué par un radical -OR⁵, ou
un radical choisi parmi les radicaux suivants R⁵, nitro, CO₂R³, -SR⁵, SO₂R⁵, SOR⁵ et -OR⁵;

7. Un procédé selon l'une quelconque des revendications précédentes dans la quelle R³ représente :
un atome d'hydrogène, ou
un radical alkyle linéaire ou ramifié, contenant de 1 à 6 atomes de carbone.

8. Un procédé selon l'une quelconque des revendications précédentes dans la quelle R⁴ représente un atome d'hydrogène.

9. Un procédé selon l'une quelconque des revendications précédentes dans la quelle R⁵ représente un radical alkyle linéaire ou ramifié, contenant de 1 à 6 atomes de carbone éventuellement substitué par un ou plusieurs atomes d'halogène.

10. Un procédé selon la revendication 1 dans laquelle :
R représente un radical -CO₂R³ ou -COR⁵;
R¹ représente :
un radical alkyle, linéaire ou ramifié, contenant de 1 à 6 atomes de carbone,
un radical cycloalkyle,contenant de 3 à 4 atomes de carbone, éventuellement substitué par un ou plusieurs substituants choisis parmi un ou plusieurs radicaux R⁵,
R² représente :
un atome d'halogène ; ou
un radical alkyle, contenant de 1 à 6 atomes de carbone, substitué par un radical -OR⁵ ou
un radical choisi parmi les radicaux suivants R⁵, un radical nitro, CO₂R³, -SR⁵, SO₂R⁵, SOR⁵, -OR⁵ ou un radical -O(CH₂)_{q}-OR⁵;
R³ représente un radical alkyle, linéaire ou ramifié, contenant de 1 à 6 atomes de carbone ;
R³¹ représente un atome d'hydrogène
R⁴ représente un atome d'hydrogène,
R⁵ représente un radical alkyle, linéaire ou ramifié, contenant de 1 à 3 atomes de carbone, éventuellement substitué par un ou plusieurs atomes d'halogènes ;
un des radicaux R² est en position ortho sur le cycle phényle ; n représente 2 ou 3.

11. Un procédé selon la revendication 1 pour la préparation d'un composé qui est le :
3-Méthyl-4-(2-nitro-4-trifluorométhylbenzoyl)-isoxazole ;
3-(1-Méthyléthyl)-4-(2-nitro-4-trifluorométhylbenzoyl)-isoxazole ;
3-Ethyl-4-(2-nitro-4-trifluorométhylbenzoyl)-isoxazole-3-carboxylate ;
3-Bromo-4-(2-nitro-4-trifluorométhylbenzoyl)-isoxazole ;
4-(2-nitro-4-trifluorométhylbenzoyl)-3-trifluorométhylisoxazole ;
5-Cyclopropyl-3-Méthyl-4-(2-nitro-4-trifluorométhylbenzoyl)-isoxazole ;
5-Méthyl-4-(2-nitro-4-trifluorométhylbenzoyl)-isoxazole-3-carboxylate d'éthyle ;
5-Méthyl-4-(2-nitro-4-trifluorométhylbenzoyl)-isoxazole-3-carboxamide ;
l) 5-Cyclopropyl-4-(2-nitro-4-trifluorométhylbenzoyl)-isoxazole-3-carboxylate d'éthyle ;
3-Cyano-5-Méthyl-4-(2-nitro-4-trifluorométhylbenzoyl)-isoxazole ;
4-(2-chloro-4-méthylsulfonylbenzoyl)-5-cyclopropylisoxazole-3-carboxylate d'éthyle ;

12. Un procédé selon la revendication 1, pour la préparation d'un composé qui est le :
5-Cyclopropyl-4-(4-méthylsulfonyl-2-trifluorométhylbenzoyl)-isoxazole-3-carboxylate d'éthyle ;
4-(2-chloro-4-méthylsulfonylbenzoyl)-5-cyclopropylisoxazole-3-carboxylate de méthyle ;
4-(2-chloro-4-méthylsulfonylbenzoyl)-5-cyclopropylisoxazole-3-carboxamide
5-Cyclopropyl-4-(2-méthylsulfonylbenzoyl)-isoxazole-3-carboxylate d'éthyle
5-Cyclopropyl-4-(4-méthysulfonyl-2-trifluorométhylbenzoyl)-isoxazole-3-carboxylate de méthyle ;
5-Cyclopropyl-4-(2-nitro-4-méthylsulfonylbenzoyl)-isoxazole-3-carboxylate d'éthyle ;
5-Cyclopropyl-4-(2,3-dichloro-4-méthylsulfonylbenzoyl)-isoxazole-3-carboxylate d'éthyle ;
4-(2-Chloro-3-méthoxy-4-méthylsulfonylbenzoyl)-5-cyclopropylisoxazole-3-carboxylate d'éthyle ;
4-(2-Chloro-4-méthylsulfonylbenzoyl)-3-cyano-5-cyclopropylisoxazole ;
4-(4-Chloro-2-méthylsulfonylbenzoyl)-5-cyclopropylisoxazole-3-carboxylate d'éthyle ;
4-(4-Chloro-2-nitrobenzoyl)-5-cyclopropylisoxazole-3-carboxylate d'éthyle
4-(4-chloro-2-trifluorométhylbenzoyl)-5-cyclopropylisoxazole-3-carboxylate d'éthyle ;
5-Cyclopropyl-4-(2-méthylsulfényl-4-trifluorométhylbenzoyl)-isoxazole-3-carboxylate d'éthyle ;
5-Cyclopropyl-4-(4-méthylsulfényl-2-trifluorométhylbenzoyl)-isoxazole-3-carboxylate d'éthyle ;
5-Cyclopropyl-4-(2-méthylsulfinyl-4-trifluorométhylbenzoyl)-isoxazole-3-carboxylate d'éthyle ;
5-Cyclopropyl-4-(4-méthylsulfinyl-2-trifluorométhylbenzoyl)-isoxazole-3-carboxylate d'éthyle ;
5-Cyclopropyl-4-(2-méthylsulfonyl-4-trifluorométhylbenzoyl)-isoxazole-3-carboxylate d'éthyle ;
5-Cyclopropyl-4-(2-fluoro-4-méthylsulfonylbenzoyl)-isoxazole-3-carboxylate d'éthyle ;
4-(2-Chloro-4-éthylsulfonylbenzoyl)-5-cyclopropylisoxazole-3-carboxylate d'éthyle ;
4-(4-Bromo-2-méthylsulfonylbenzoyl)-5-cyclopropylisoxazole-3-carboxylate d'éthyle ;
4-(2-Bromo-4-méthylsulfonylbenzoyl)-5-cyclopropylisoxazole-3-carboxylate d'éthyle ;
4-(2-Choro-4-méthylsulfénylbenzoyl)-5-cyclopropylisoxazole-3-carboxylate d'éthyle ;
4-(2-Choro-4-méthylsulfinylbenzoyl)-5-cyclopropylisoxazole-3-carboxylate d'éthyle ;
3-Acétyl-4-(2-Chloro-4-méthylsulfonylbenzoyl)-5-cyclopropylisoxazole
4-(2-Chloro-4-méthylsulfonylbenzoyl)-5-cyclopropylisoxazole-3-carboxylate d'isopropyle ;
4-(4-Chloro-2-méthylsulfonylbenzoyl)-5-cyclopropylisoxazole-3-carboxylate de méthyle ;
5-Cyclopropyl-4-(2-méthylsulfonyl-4-trifluorométhylbenzoyl)-isoxazole-3-carboxylate de méthyle ;
4-(4-Chloro-2-méthylsulfénylbenzoyl)-5-cyclopropylisoxazole-3-carboxylate de méthyle ;
4-(4-Chloro-2-méthylsulfinylbenzoyl)-5-cyclopropylisoxazole-3-carboxylate de méthyle ;

13. Un procédé selon la revendication 1 pour la préparation d'un composé qui est le :
5-Cyclopropyl-4-(2-méthylsulfonyl-4-trifluorométhylbenzoyl)-isoxazole-3-carboxylate d'éthyle.

14. Un procédé pour la préparation d'une composition herbicide qui consiste à formuler, comme matière active, une quantité herbicide efficace d'un dérivé du 4-benzoyl isoxazole de formule générale I tel que défini dans la revendication 1 dans laquelle :
R représente:,
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle,contenant de 3 à 6 atomes de carbone, éventuellement substitué par un ou plusieurs substituants choisis parmi les radicaux R⁵, un ou plusieurs atomes d'halogènes et un radical CO₂R₃ ; ou
un radical choisi parmi les radicaux suivants: CO₂R³, -COR⁵, cyano, nitro, -CONR³¹R⁴, ou
un atome d'halogène ;
R¹ représente :
Un atome d'hydrogène, ou
un radical alkyle, alkényle ou alkynyle linéaire ou ramifié, contenant de 1 à 6 atomes de carbone, éventuellement substitué par un ou plusieurs atomes d'halogènes ; ou
un radical cycloalkyle,contenant de 3 à 6 atomes de carbone, éventuellement substitué par un ou plusieurs substituants choisis parmi les radicaux R⁵ et un ou plusieurs atomes d'halogènes ;
R² représente :
un atome d'halogène, ou
Un radical choisi parmi les radicaux suivants : R⁵, -SR⁵, SOR⁵ SO₂R⁵, -SO₂NR³¹R⁴, -CO₂R³, -COR⁵, CONR³¹R⁴, CSNR³¹R⁴, -OR⁵, un radical nitro, un radical cyano, un radical -O(CH₂)_{q}-OR⁵, ou un radical alkyle, linéaire ou ramifié, contenant de 1 à 6 atomes de carbone, et qui est substitué par un radical OR⁵ ;
R³, R³¹ et R⁴, qui peuvent être identiques ou différents, représentent chacun
un atome d'hydrogène, ou
un radical alkyle, alkényle ou alkynyle linéaire ou ramifié, contenant de 1 à 6 atomes de carbone, éventuellement substitué par un ou plusieurs atomes d'halogènes ; ou
R⁵ représente :
un radical alkyle, alkényle ou alkynyle linéaire ou ramifié, contenant de 1 à 6 atomes de carbone, éventuellement substitué par un ou plusieurs atomes d'halogènes ;
n représente un nombre entier pouvant prendre toutes les valeurs de 1 à 5 ;
q représente un nombre entier pouvant prendre toutes les valeurs de 1 à 3 ;
à condition que R et R¹ ne représentent pas simultanément un radical méthyle ;
ou les sels de ces composés acceptables pour des usages agricoles, associé avec des diluants ou des charges acceptables pour des applications herbicides et/ou des agents tensio-actifs acceptables pour des applications herbicides.

15. Un procédé pour la préparation d'une composition herbicide qui consiste à formuler, comme matière active, une quantité herbicide efficace d'un composé selon l'une quelconque des revendications 1 à 13, associée avec des diluants ou des charges acceptables pour des applications herbicides et/ou des agents tensio-actifs acceptables pour des applications herbicides.

16. Un procédé selon la revendication 14 ou 15 dans lequel la composition herbicide contient de 0,05 à 90% en poids de la matière active.

17. Un procédé selon l'une quelconque des revendications 14 à 16 dans lequel la composition herbicide se présente sous forme liquide et contient de 0,05 à 25% en poids d'un agent tensioactif.

18. Un procédé selon l'une quelconque des revendications 14 à 16 dans lequel la composition herbicide se présente sous forme d'une suspension aqueuse concentrée ou d'une poudre mouillable ou d'une poudre soluble dans l'eau ou d'une poudre dispersible dans l'eau, ou d'un concentré liquide soluble dans l'eau ou dispersible dans l'eau ou d'une suspension liquide émulsionnable ou d'un granulé.

19. Un procédé selon la revendication 18 dans lequel la composition herbicide se présente sous forme d'une suspension aqueuse contenant de 10 à 70% de matière active, ou d'une poudre mouillable contenant de 10 à 90% de matière active, ou d'une poudre soluble dans l'eau ou d'une poudre dispersible dans l'eau contenant de 10 à 90% de matière active, ou d'un concentré liquide soluble dans l'eau ou dispersible dans l'eau contenant de 5 à 50% de matière active, ou d'une suspension liquide concentrée émulsionnable contenant de 10 à 70% de matière active ou de granulés contenant de 1 à 90% de matière active, les pourcentages étant donnés en poids.

20. Un procédé selon la revendication 18 ou 19 dans laquelle la composition herbicide se présente sous forme d'une suspension aqueuse contenant de 2 à 10% d'un agent tensioactif, ou d'une poudre mouillable contenant de 2 à 10% d'un agent tensioactif, ou d'une suspension liquide concentrée émulsionnable contenant de 5 à 15% d'un agent tensioactif, ou d'un concentré liquide soluble dans l'eau contenant de 5 à 25% d'un agent tensioactif, ou de granulés contenant de 0,5 à 7%d'un agent tensioactif, les pourcentages étant donnés en poids.

21. Un procédé de contrôle de la croissance des mauvaises herbes dans un lieu consistant à appliquer sur le dit-lieu une quantité herbicide efficace d'un dérivé du 4-benzoyl isoxazole selon l'une quelconque des revendications 1 à 14, ou d'un sel acceptable pour des applications agricoles, de ce composé.

22. Un procédé selon la revendication 21 dans lequel le lieu est une zone utilisée ou destinée à être utilisée pour y faire pousser des cultures, et où la dose d'emploi utilisée est comprise entre 0,01 et 4 kg de matière active par hectare.

23. Un procédé selon la revendication 21 dans lequel le lieu est une zone de non-culture et où la dose d'application est comprise entre 1,0 et 20 kg de matière active par hectare.

24. Un dérivé du 4-benzoyl isoxazole tel que défini dans l'une quelconque des revendications 1 à 14.

25. Une composition herbicide telle que définie dans l'une quelconque des revendications 14 à 20.
